# EUROPEAN PATENT APPLICATION

(11) **EP 4 706 670 A1**
(43) Date of publication of application: **11.03.2026**
(21) Application number: 24796195.6
(22) Date of filing: 25.04.2024
(51) Int. Cl.: A61K 38/08, C07K 7/08, A61K 47/64, A61P 25/00, A61P 25/08, A61P 9/10

(54) **PSD-95 DOMAIN INHIBITOR, AND PREPARATION METHOD THEREFOR AND USE THEREOF**

(30) Priority: 26.04.2023 CN 202310478122; 12.05.2023 CN 202310538729; 26.05.2023 CN 202310610253; 02.06.2023 CN 202310653468; 12.06.2023 CN 202310692754; 28.07.2023 CN 202310944465; 28.07.2023 CN 202310942918; 05.09.2023 CN 202311140287; 13.09.2023 CN 202311185290
(71) Applicant: Shenzhen Salubris Pharmaceuticals Co., Ltd., Shenzhen, Guangdong 518017 (CN)
(72) Inventor: XU, Wenjie, Shenzhen, Guangdong 518017 (CN); LIAN, Xiaolei, Shenzhen, Guangdong 518017 (CN); ZENG, Junnan, Shenzhen, Guangdong 518017 (CN); XIAO, Ying, Shenzhen, Guangdong 518017 (CN); HUA, Huaijie, Shenzhen, Guangdong 518017 (CN); LI, Qinze, Shenzhen, Guangdong 518017 (CN); ZHANG, Ying, Shenzhen, Guangdong 518017 (CN); CAO, Dapeng, Shenzhen, Guangdong 518017 (CN)
(74) Representative: Biggi, Cristina
(86) International application number: PCT/CN2024/089903
(87) International publication number: WO 2024/222819

(57) **Abstract**

A PSD-95 domain inhibitor, a preparation method therefor and the use thereof, a pharmaceutical composition containing the compounds, and the use of the compound in the preparation a drug for treating a disease.

(TAT)m-L₁-(L₂)n I

## Description

### FIELD OF THE INVENTION

The present invention belongs to the technical field of chemical drug, and specifically relates to PSD-95 domain inhibitor, preparation method therefor and use thereof.

### BACKGROUND OF THE INVENTION

Postsynaptic density protein-95 (PSD-95) is a protein encoded by the DLG4 (disks large homolog 4) gene in humans. PSD-95 conprises three PDZ domains (PDZ1, PDZ2, and PDZ3), an SH3 domain, and a guanylate kinase-like domain (GK) connected by a linker region. It is located almost exclusively in the postsynaptic density of neurons and is involved in anchoring synaptic proteins. Its direct and indirect binding partners include neuroligins, neuronal nitric oxide synthase (nNOS), N-methyl-d-aspartate (NMDA) receptors, AMPA receptors, and potassium channels.

PSD-95 binds to NMDA receptors (primarily the GluN2A and GluN2B subunits) and nNOS via the PDZ domains. Activation of NMDA receptors leads to calcium ion influx, which activates nNOS, thereby producing nitric oxide (NO). Therefore, PSD-95 mediates a specific link between activation of NMDA receptors and NO production. It could be detrimental to cells if NO production continues for a prolonged period. Currently, targeted inhibition of PSD-95 to block the formation of nNOS/PSD-95/NMDA receptor ternary complex, thereby reducing the release of NO, has become a research focus. For example, Patent CN106661126B discloses a PSD-95 inhibitor NA-1, which can be used in the treatment of stroke, subarachnoid hemorrhage, or CNS trauma, and the like, through the blockade of PSD-95.

Patent WO2022150655A1 describes PSD-95 domain inhibitors for improving therapeutic index. By employing D-amino acids to replace the L-amino acids in NA-1, plasmin-resistance can be conferred on these inhibitors, significantly improving stability of the inhibitors without nephrotoxicity.

Although PSD-95 domain inhibitors have been reported in the prior art, there are no marketed products to date. Furthermore, the PSD-95 domain inhibitors in the prior art suffer from problems such as poor stability, short half-life, and low activity. Consequently, there is an urgent need to develop more PSD-95 domain inhibitors.

### SUMMARY OF THE INVENTION

In view of the problems of the prior art, the present application provides a PSD-95 domain inhibitor, preparation method therfor, and use thereof.

Specifically, the present invention provides a PSD-95 domain inhibitor, characterized by having a structure shown in formula (I):

(TAT)m-L₁-(L₂)n (Formula I);

wherein, the TAT is selected from the group consisting of YGrKKRrQrR, YGrKKRrErR, YGrKKRrErRR, YGrKKRrQrRR, YGR(Me)KKRR(Me)QR(Me)RR, YGR(Me)₂KKRR(Me)₂QR(Me)₂RR, YGR(Me)KKRrQrRR, YGrKKRR(Me)QrRR, YGrKKRrQR(Me)RR, YGR(Me)KKRrQR(Me)RR, YGrKKRR(Me)QR(Me)RR, YGR(Me)KKRR(Me)QrRR, YGr(Me)KKRrQrRR, YGrKKRrQr(Me)RR, YGrKKRr(Me)QrRR, YGr(Me)KKRr(Me)QrRR, YGr(Me)KKRrQr(Me)RR, YGrKKRr(Me)Qr(Me)RR, YGr(Me)KKRr(Me)Qr(Me)RR, YGR(Et)KKRR(Et)QR(Et)RR, YGRKKRrErRR, YGR(Me)KKRrErRR, YGrKKRrERRr, YGR(Ac)KKRR(Ac)QR(Ac)RR, YGR(Ac)₂KKRR(A_{c})₂QR(Ac)₂RR, YGR(Ac)KKRrQrRR, YGR*KKRRQRRR, YGR*KKRrQrRR, YGR*KKRrErRR, YGRKKRR*QRRR, YGrKKRR*QrRR, YGRKKRRQR*RR, YGrKKRrQR*RR, YGR*KKRR*QRRR, YGR*KKRR*QrRR, YGRKKRR*QR*RR, YGrKKRR*QR*RR, YGR*KKRRQR*RR, YGR*KKRrQR*RR, YGR*KKRR*QR*RR, YGrKKRrER(Me)RR, YGrKKRrQR(Me)RR(Me), YGrKKRrQRRR, YGR*KKRrQR(Me)RR and YGR*KKRrER(Me)RR, wherein 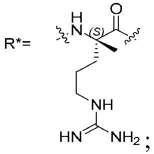
the L₁ is selected from the group consisting of KLSS, K(Me)LSS, kLSS, 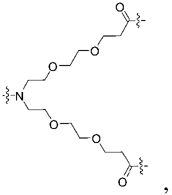 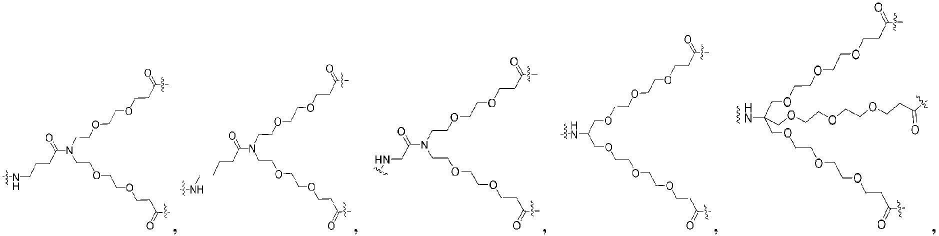 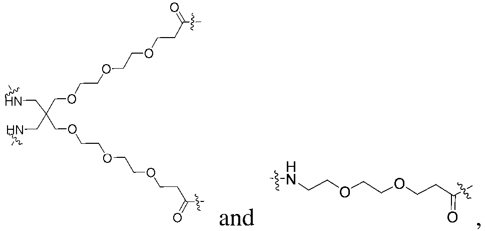 wherein the straight line perpendicular to the wavy line denotes a connection site; when TAT is YGrKKRrQrRR, L₁ is 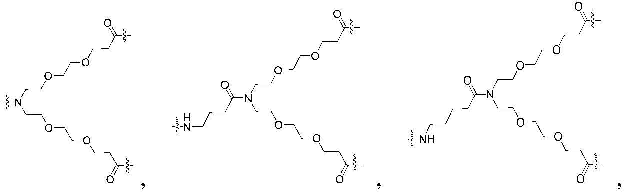 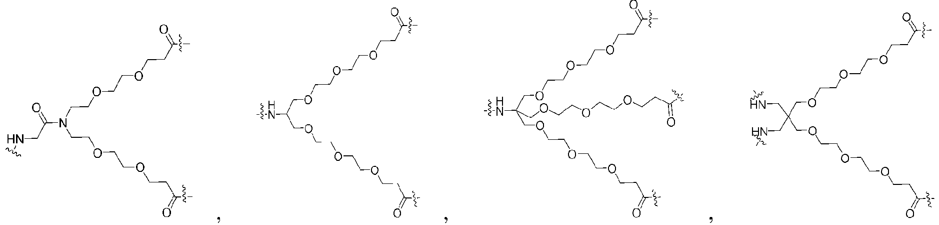 or 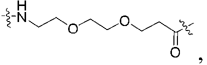 wherein the straight line perpendicular to the wavy line denotes a connection site;
the L₂ is selected from the group consisting of IETDV, IETAV, IESDV, KETLV, KETTV, ISTDV and VETVV;
the m and n are each independently selected from the group consisting of the integers of 1, 2 and 3;
for amino acids in the TAT, L₁, and L₂, an uppercase letter denotes an L-amino acid, lowercase letter denotes a D-amino acid, Me is methyl, Et is ethyl, and Ac is acetyl.

Further, as a preferred technical solution of the present invention, the TAT is selected from the group consisting of YGR(Me)KKRR(Me)QR(Me)RR, YGR(Me)₂KKRR(Me)₂QR(Me)₂RR, YGR(Me)KKRrQrRR, YGrKKRR(Me)QrRR, YGrKKRrQR(Me)RR, YGR(Me)KKRrQR(Me)RR, YGrKKRR(Me)QR(Me)RR, YGR(Me)KKRR(Me)QrRR, YGr(Me)KKRrQrRR, YGrKKRrQr(Me)RR, YGrKKRr(Me)QrRR, YGr(Me)KKRr(Me)QrRR, YGr(Me)KKRrQr(Me)RR, YGrKKRr(Me)Qr(Me)RR, YGr(Me)KKRr(Me)Qr(Me)RR, YGR(Et)KKRR(Et)QR(Et)RR, YGRKKRrErRR, YGR(Me)KKRrErRR, YGrKKRrERRr, YGR(Ac)KKRR(Ac)QR(Ac)RR, YGR(Ac)₂KKRR(Ac)_{2Q}R(Ac)₂RR, YGR(Ac)KKRrQrRR, YGR*KKRRQRRR, YGR*KKRrQrRR, YGR*KKRrErRR, YGRKKRR*QRRR, YGrKKRR*QrRR, YGRKKRRQR*RR, YGrKKRrQR*RR, YGR*KKRR*QRRR, YGR*KKRR*QrRR, YGRKKRR*QR*RR, YGrKKRR*QR*RR, YGR*KKRRQR*RR, YGR*KKRrQR*RR, YGR*KKRR*QR*RR, YGrKKRrER(Me)RR, YGrKKRrQR(Me)RR(Me), YGrKKRrQRRR, YGR*KKRrQR(Me)RR and YGR*KKRrER(Me)RR, wherein 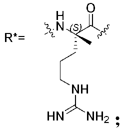
the L₁ is selected from the group consisting of KLSS, K(Me)LSS, kLSS, 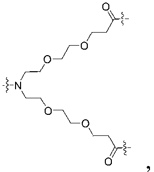 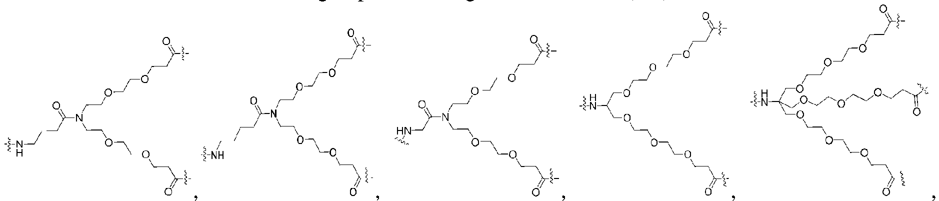 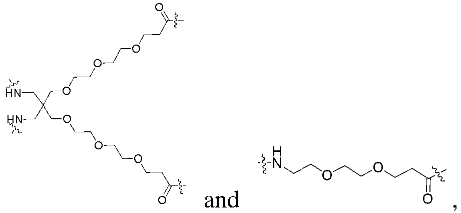 wherein the straight line perpendicular to the wavy line denotes a connection site;
the L₂ is selected from the group consisting of IETDV, IETAV, IESDV, KETLV, KETTV, ISTDV and VETVV;
the m and n are each independently selected from the group consisting of the integers of 1, 2 and 3;
for amino acids in the TAT, L₁, and L₂, an uppercase letter denotes an L-amino acid, lowercase letter denotes a D-amino acid, Me is methyl, Et is ethyl, and Ac is acetyl.

As a preferred enbodiment of the present invention, the TAT is a cell-penetrating peptide, and the TAT is selected from the group consisting of YGR(Me)KKRR(Me)QR(Me)RR, YGR(Me)₂KKRR(Me)₂QR(Me)₂RR, YGR(Me)KKRrQrRR, YGrKKRR(Me)QrRR, YGrKKRrQR(Me)RR, YGR(Me)KKRrQR(Me)RR, YGrKKRR(Me)QR(Me)RR, YGR(Me)KKRR(Me)QrRR, YGr(Me)KKRrQrRR, YGrKKRrQr(Me)RR, YGrKKRr(Me)QrRR, YGr(Me)KKRr(Me)QrRR, YGr(Me)KKRrQr(Me)RR, YGrKKRr(Me)Qr(Me)RR, YGr(Me)KKRr(Me)Qr(Me)RR, YGR(Et)KKRR(Et)QR(Et)RR, YGRKKRrErRR, YGR(Me)KKRrErRR, YGrKKRrERRr, YGR(Ac)KKRR(Ac)QR(Ac)RR, YGR(Ac)₂KKRR(Ac)_{2Q}R(Ac)₂RR, YGR(Ac)KKRrQrRR, YGR*KKRRQRRR, YGR*KKRrQrRR, YGR*KKRrErRR, YGRKKRR*QRRR, YGrKKRR*QrRR, YGRKKRRQR*RR, YGrKKRrQR*RR, YGR*KKRR*QRRR, YGR*KKRR*QrRR, YGRKKRR*QR*RR, YGrKKRR*QR*RR, YGR*KKRRQR*RR, YGR*KKRrQR*RR and YGR*KKRR*QR*RR, wherein 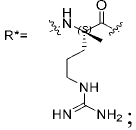
the L₁ is selected from the group consisting of KLSS, kLSS, 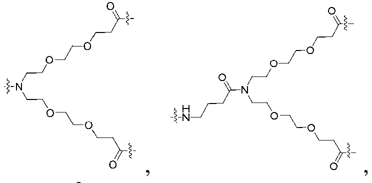 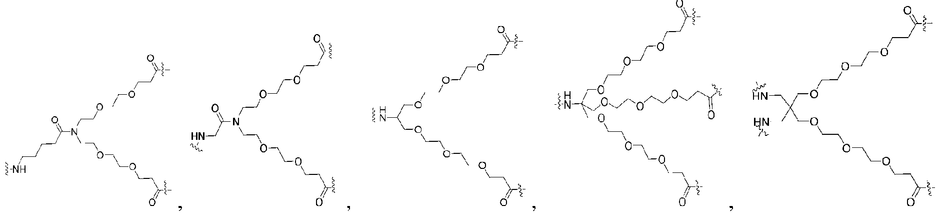 and 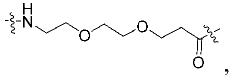 wherein the straight line perpendicular to the wavy line denotes a connection site;
the L₂ is selected from the group consisting of IETDV, IETAV, IESDV, KETLV, KETTV, ISTDV and VETVV;
the m and n are each independently selected from the group consisting of the integers of 1, 2 and 3.

As a preferred embodiment of the present invention, the L₁ is selected from the group consisting of kLSS,

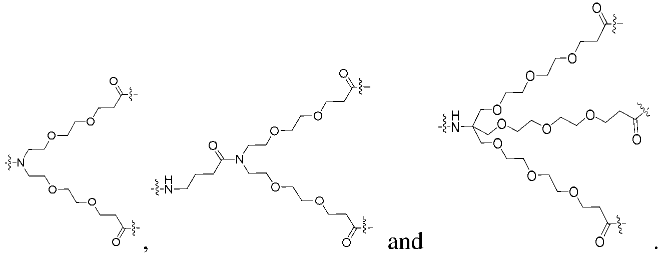

As a preferred embodiment of the present invention, the L₂ is selected from the group consisting of IETDV and ISTDV.

As a preferred embodiment of the present invention, the PSD-95 domain inhibitor is selected from a structure shown in Table A, Table B, Table C, Table D, Table E, Table F, Table G, Table H, Table I or

**Table J:**

| No. | Compound | No. | Compound |
|---|---|---|---|
| A1 | YGR(Me)KKRR(Me)QR(Me)RRKLSSI ESDV | A2 | YGR(Me)KKRR(Me)QR(Me)RRkL SSIETDV |
| A3 | 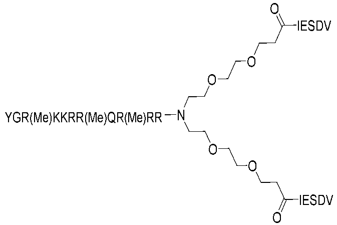 | A4 | 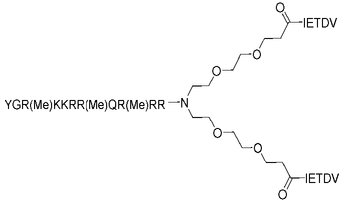 |
| A5 | 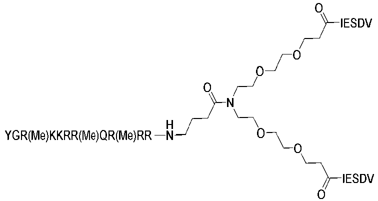 | A6 | 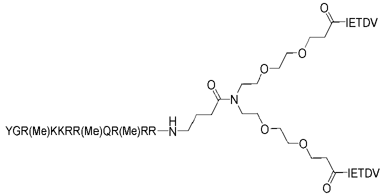 |
| A7 | 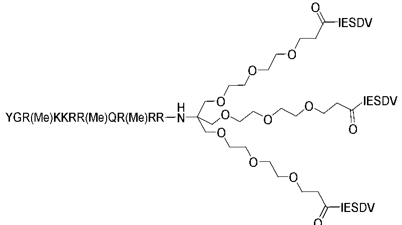 | A8 | 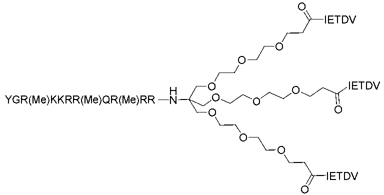 |

**Table B**

| No. | Compound | No. | Compound |
|---|---|---|---|
| B1 | YGR(Me)₂KKRR(Me)₂QR(Me)₂RRkL SSIESDV | B2 | YGR(Me)₂KKRR(Me)₂QR(Me)₂RR kLSSIETDV |
| B3 | 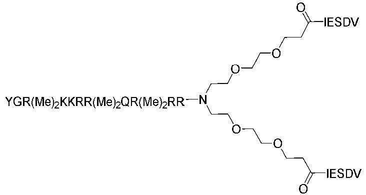 | B4 | 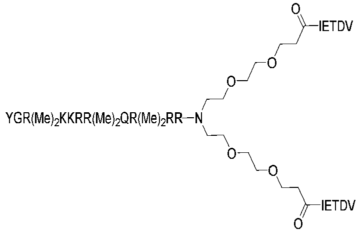 |
| B5 | 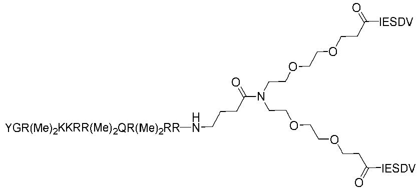 | B6 | 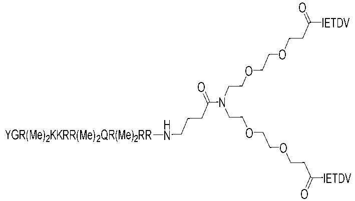 |
| B7 | 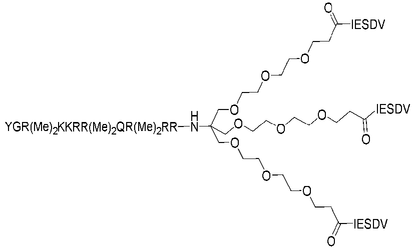 | B8 | 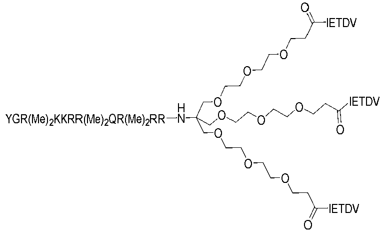 |

**Table C**

| No. | Compound | No. | Compound |
|---|---|---|---|
| C1 | YGR(Me)KKRrQrRRkLSSIESDV | C2 | YGR(Me)KKRrQrRRkLSSIETDV |
| C3 | 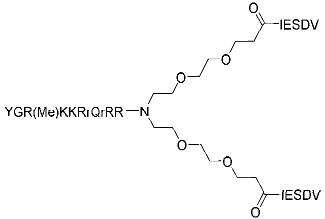 | C4 | 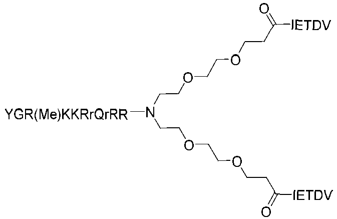 |
| C5 | 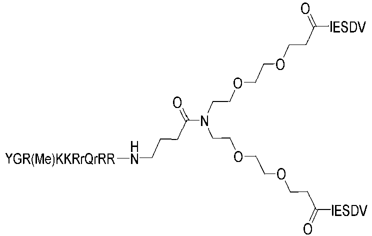 | C6 | 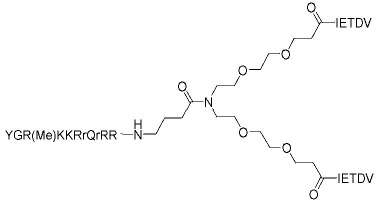 |
| C7 | 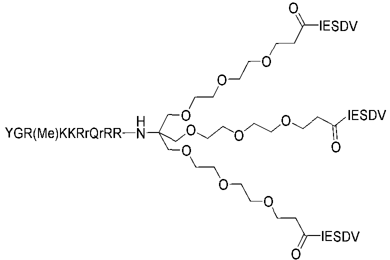 | C8 | 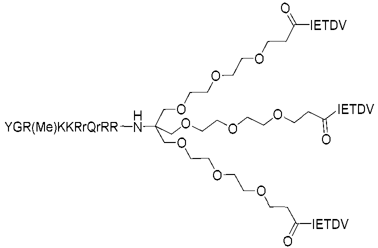 |

**Table D**

| No. | Compound | No. | Compound |
|---|---|---|---|
| D1 | YGR(Ac)KKRR(Ac)QR(Ac)RRkLSSI ESDV | D2 | YGR(Ac)KKRR(Ac)QR(Ac)RRkLS SIETDV |
| D3 | 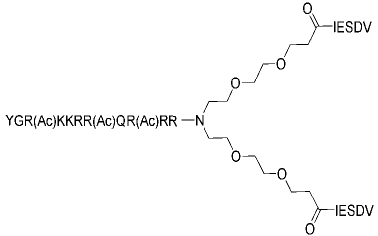 | D4 | 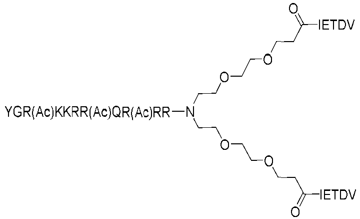 |
| D5 | 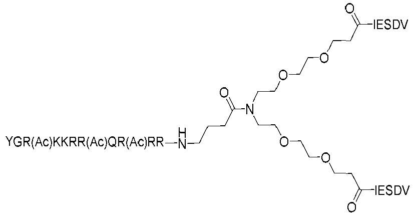 | D6 | 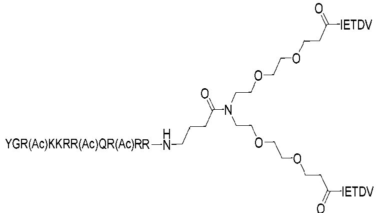 |
| D7 | 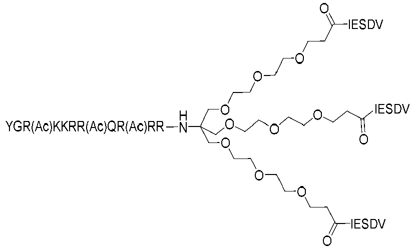 | D8 | 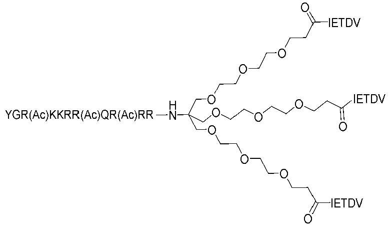 |

**Table E**

| No. | Compound | No. | Compound |
|---|---|---|---|
| E1 | YGR(Ac)₂KKRR(Ac)₂QR(Ac)₂RRkLS SIESDV | E2 | YGR(Ac)₂KKRR(Ac)₂QR(Ac)₂RRk LSSIETDV |
| E3 | 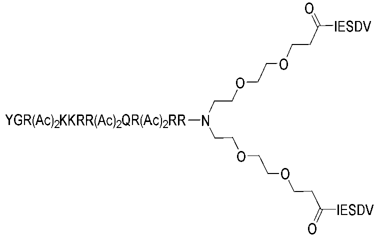 | E4 | 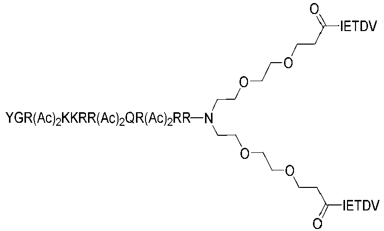 |
| E5 | 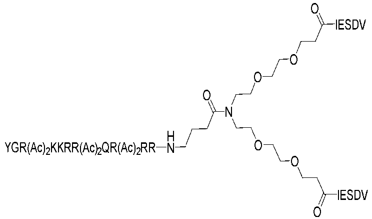 | E6 | 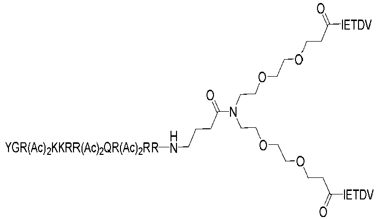 |
| E7 | 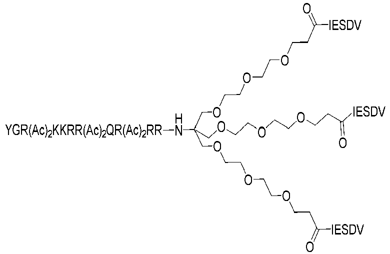 | E8 | 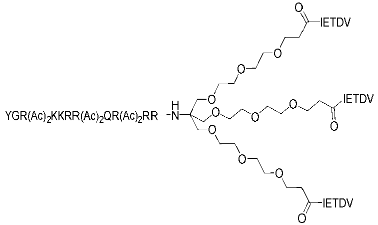 |

**Table F**

| No. | Compound | No. | Compound |
|---|---|---|---|
| F1 | YGR(Ac)KKRrQrRRkLSSIESDV | F2 | YGR(Ac)KKRrQrRRkLSSIETDV |
| F3 | 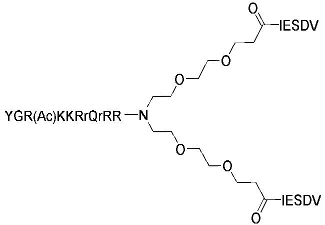 | F4 | 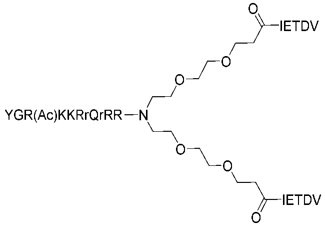 |
| F5 | 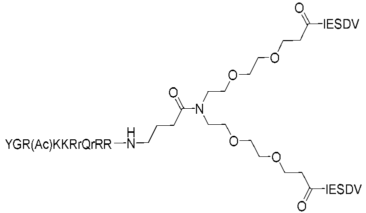 | F6 | 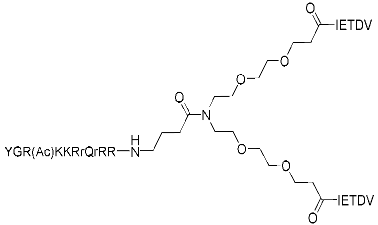 |
| F7 | 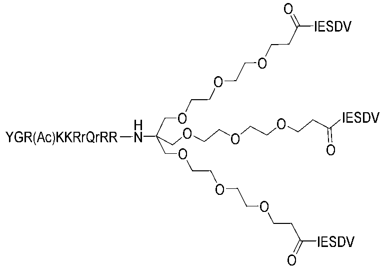 | F8 | 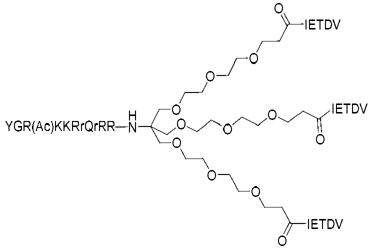 |

**Table G**

| No. | Compound | No. | Compound |
|---|---|---|---|
| G1 | YGR*KKRRQRRRKLSSIESDV | G2 | YGR*KKRRQRRRKLSSIETDV |
| G3 | YGR*KKRrQrRRkLSSIESDV | G4 | YGR*KKRrQrRRkLSSIETDV |
| G5 | YGRKKRR*QRRRKLSSIESDV | G6 | YGRKKRR*QRRRKLSSIETDV |
| G7 | YGrKKRR*QrRRkLSSIESDV | G8 | YGrKKRR*QrRRkLSSIETDV |
| G9 | YGRKKRRQR*RRKLSSIESDV | G10 | YGRKKRRQR*RRKLSSIETDV |
| G11 | YGrKKRrQR*RRkLSSIESDV | G12 | YGrKKRrQR*RRkLSSIETDV |
| G13 | YGR*KKRR*QRRRKLSSIESDV | G14 | YGR*KKRR*QRRRKLSSIETDV |
| G15 | YGR*KKRR*QrRRkLSSIESDV | G16 | YGR*KKRR*QrRRkLSSIETDV |
| G17 | YGRKKRR*QR*RRKLSSIESDV | G18 | YGRKKRR*QR*RRKLSSIETDV |
| G19 | YGrKKRR*QR*RRkLSSIESDV | G20 | YGrKKRR*QR*RRkLSSIETDV |
| G21 | YGR*KKRRQR*RRKLSSIESDV | G22 | YGR*KKRRQR*RRKLSSIETDV |
| G23 | YGR*KKRrQR*RRkLSSIESDV | G24 | YGR*KKRrQR*RRkLSSIETDV |
| G25 | YGR*KKRR*QR*RRkLSSIESDV | G26 | YGR*KKRR*QR*RRkLSSIETDV |
| G27 | 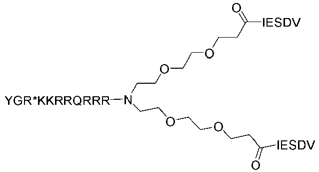 | G28 | 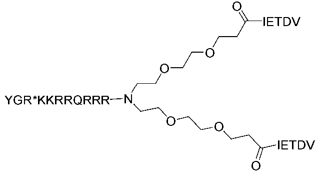 |
| G29 | 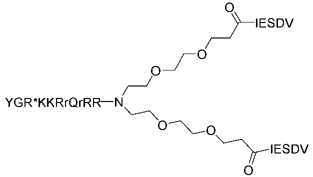 | G30 | 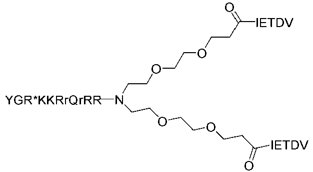 |
| G31 | 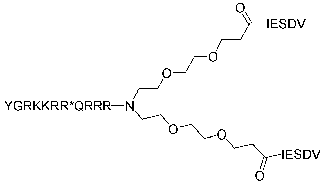 | G32 | 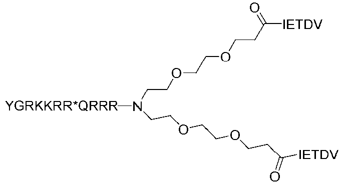 |
| G33 | 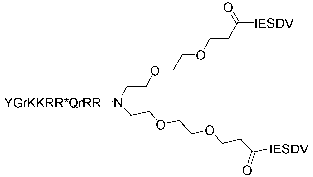 | G34 | 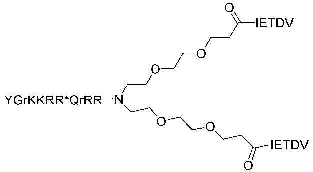 |
| G35 | 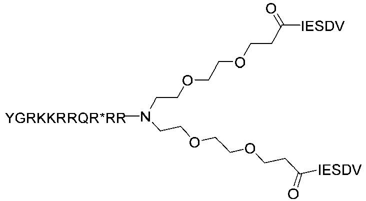 | G36 | 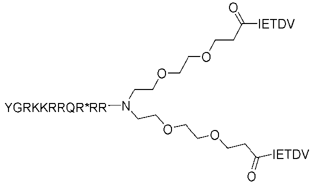 |
| G37 | 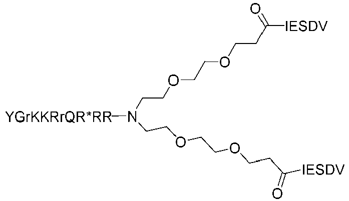 | G38 | 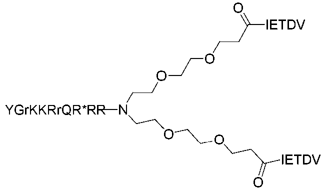 |
| G39 | 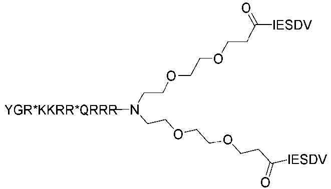 | G40 | 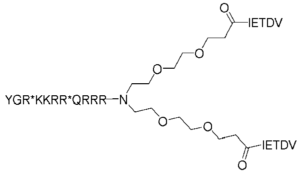 |
| G41 | 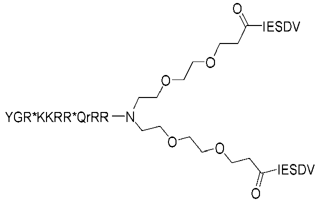 | G42 | 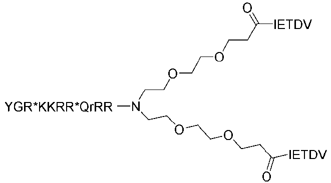 |
| G43 | 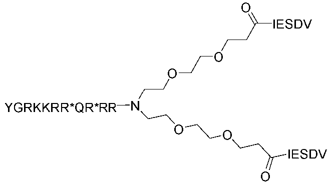 | G44 | 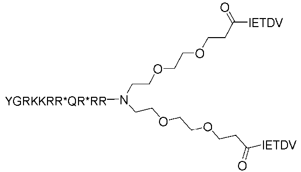 |
| G45 | 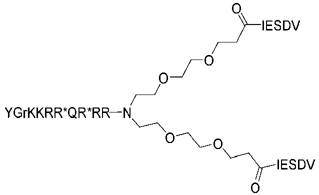 | G46 | 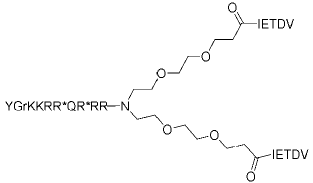 |
| G47 | 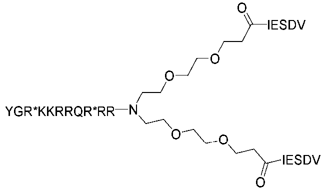 | G48 | 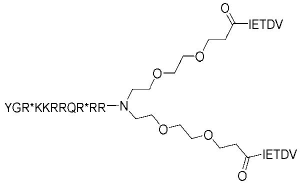 |
| G49 | 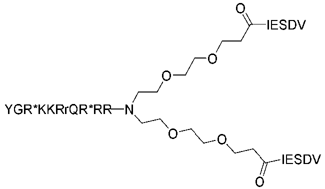 | G50 | 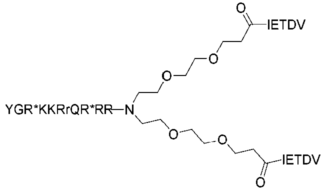 |
| G51 | 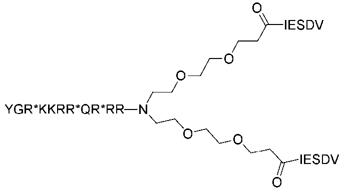 | G52 | 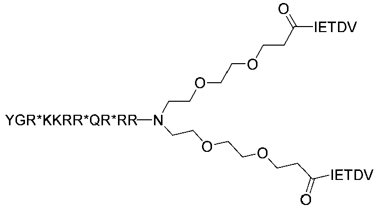 |
| G53 | 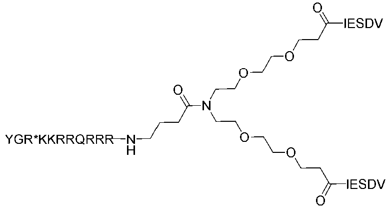 | G54 | 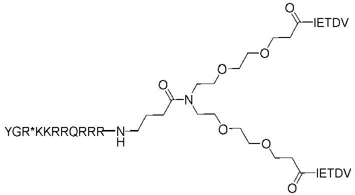 |
| G55 | 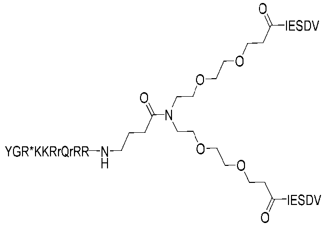 | G56 | 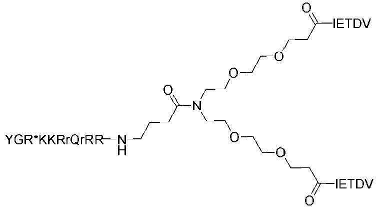 |
| G57 | 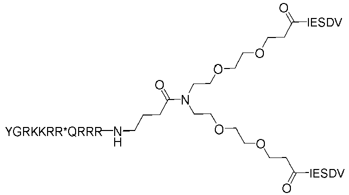 | G58 | 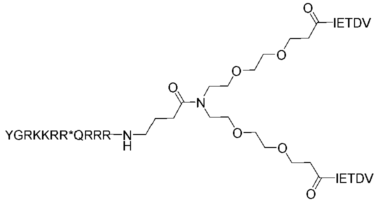 |
| G59 | 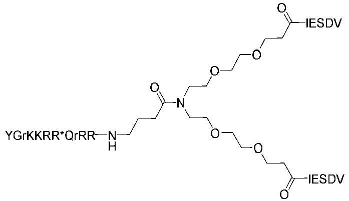 | G60 | 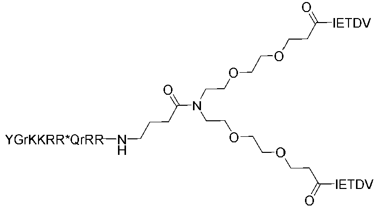 |
| G61 | 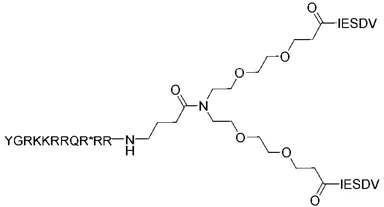 | G62 | 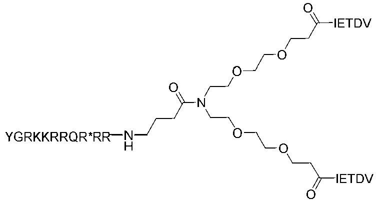 |
| G63 | 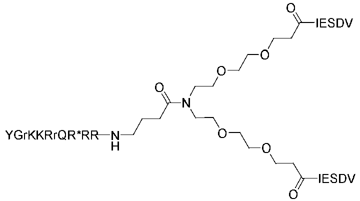 | G64 | 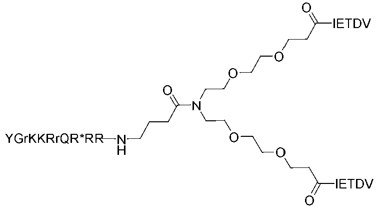 |
| G65 | 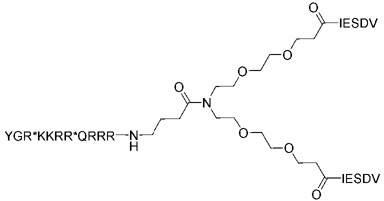 | G66 | 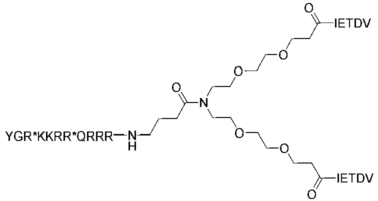 |
| G67 | 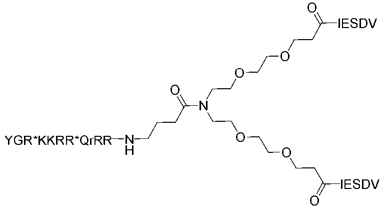 | G68 | 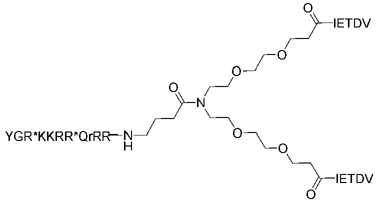 |
| G69 | 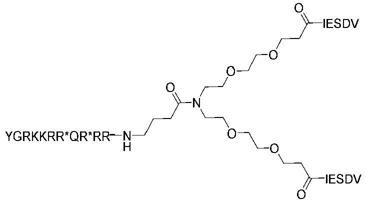 | G70 | 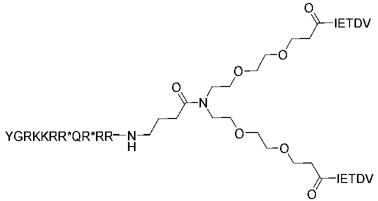 |
| G71 | 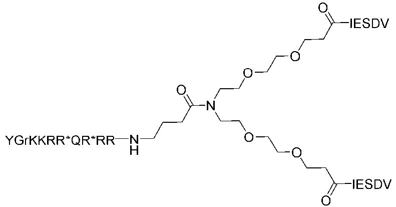 | G72 | 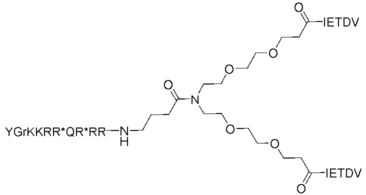 |
| G73 | 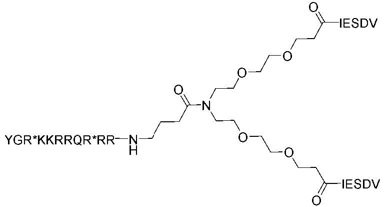 | G74 | 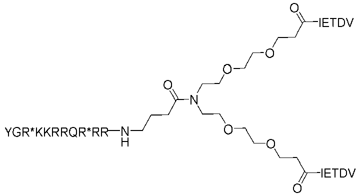 |
| G75 | 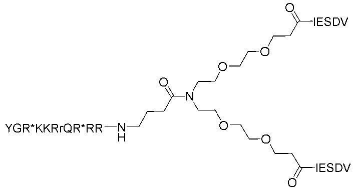 | G76 | 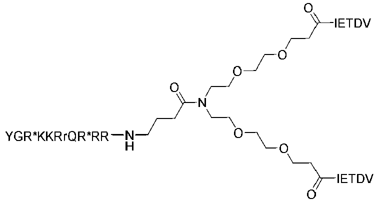 |
| G77 | 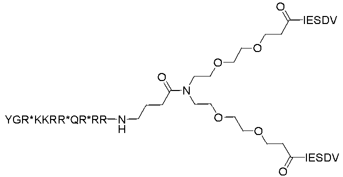 | G78 | 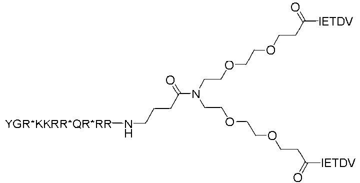 |

**Table H**

| No. | Compound | No. | Compound |
|---|---|---|---|
| H1 | YGrKKRR(Me)QrRRKLSSIESDV | H2 | YGrKKRR(Me)QrRRKLSSIETDV |
| H3 | YGrKKRrQR(Me)RRkLSSIESDV | H4 | YGrKKRrQR(Me)RRkLSSIETDV |
| H5 | YGR(Me)KKRrQR(Me)RRkLSSIESD V | H6 | YGR(Me)KKRrQR(Me)RRkLSSIETD V |
| H7 | YGrKKRR(Me)QR(Me)RRkLSSIESD V | H8 | YGrKKRR(Me)QR(Me)RRkLSSIETD V |
| H9 | YGR(Me)KKRR(Me)QrRRkLSSIESD V | H10 | YGR(Me)KKRR(Me)QrRRkLSSIETD V |
| H11 | YGr(Me)KKRrQrRRkLSSIESDV | H12 | YGr(Me)KKRrQrRRkLSSIETDV |
| H13 | YGrKKRrQr(Me)RRkLSSIESDV | H14 | YGrKKRrQr(Me)RRkLSSIETDV |
| H15 | YGrKKRr(Me)QrRRkLSSIESDV | H16 | YGrKKRr(Me)QrRRkLSSIETDV |
| H17 | YGr(Me)KKRr(Me)QrRRkLSSIESDV | H18 | YGr(Me)KKRr(Me)QrRRkLSSIETDV |
| H19 | YGr(Me)KKRrQr(Me)RRkLSSIESDV | H20 | YGr(Me)KKRrQr(Me)RRkLSSIETDV |
| H21 | YGrKKRr(Me)Qr(Me)RRkLSSIESDV | H22 | YGrKKRr(Me)Qr(Me)RRkLSSIETDV |
| H23 | YGr(Me)KKRr(Me)Qr(Me)RRkLSSIE SDV | H24 | YGr(Me)KKRr(Me)Qr(Me)RRkLSSIE TDV |
| H25 | YGR(Et)KKRR(Et)QR(Et)RRkLSSIES DV | H26 | YGR(Et)KKRR(Et)QR(Et)RRkLSSIET DV |
| H27 | YGRKKRrErRRkLSSIESDV | H28 | YGRKKRrErRRkLSSIETDV |
| H29 | YGR(Me)KKRrErRRkLSSIESDV | H30 | YGR(Me)KKRrErRRkLSSIETDV |
| H31 | YGrKKRrERRrkLSSIESDV | H32 | YGrKKRrERRrkLSSIETDV |

| H33 | YGR*KKRrErRRkLSSIESDV | H34 | YGR*KKRrErRRkLSSIETDV |
|---|---|---|---|
| H35 | 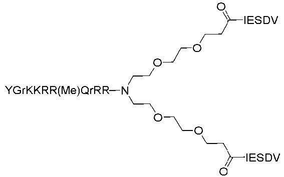 | H36 | 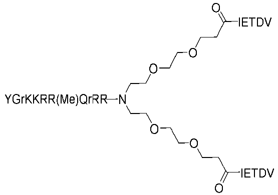 |
| H37 | 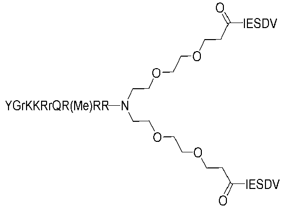 | H38 | 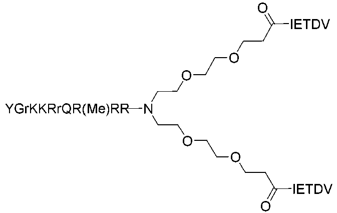 |
| H39 | 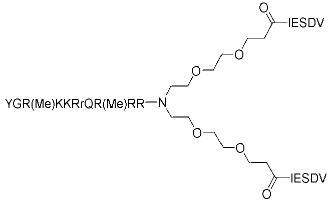 | H40 | 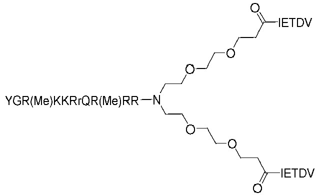 |
| H41 | 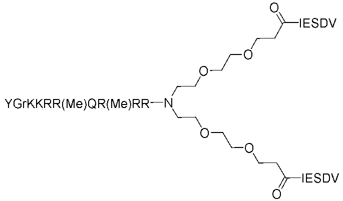 | H42 | 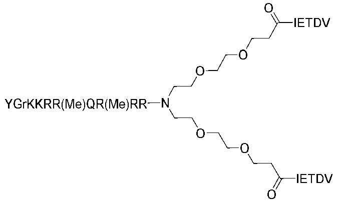 |
| H43 | 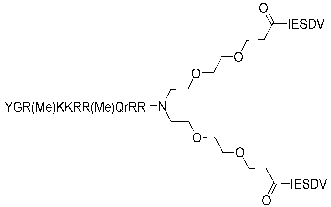 | H44 | 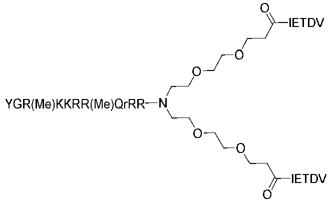 |
| H45 | 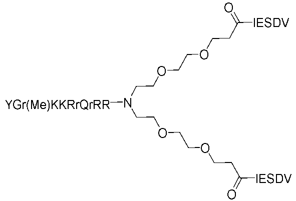 | H46 | 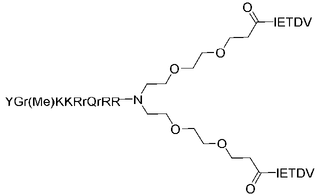 |
| H47 | 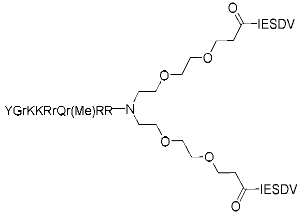 | H48 | 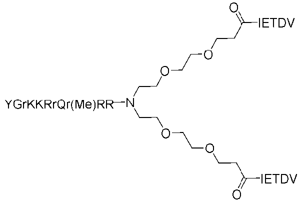 |
| H49 | 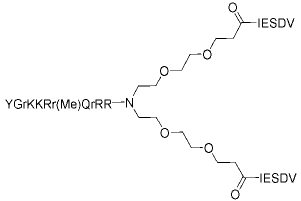 | H50 | 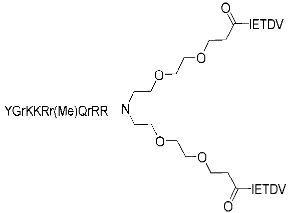 |
| H51 | 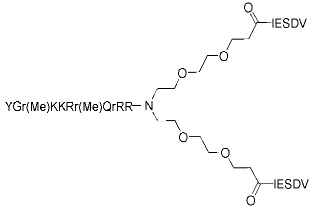 | H52 | 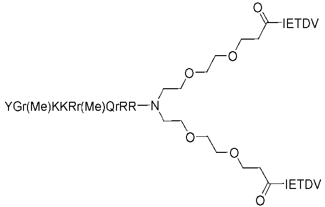 |
| H53 | 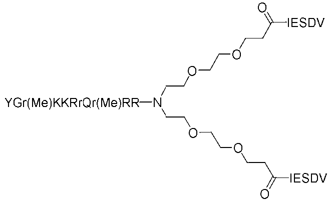 | H54 | 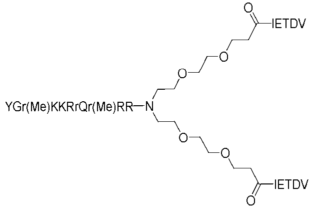 |
| H55 | 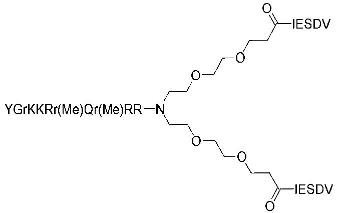 | H56 | 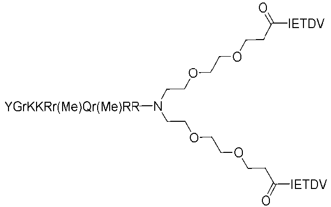 |
| H57 | 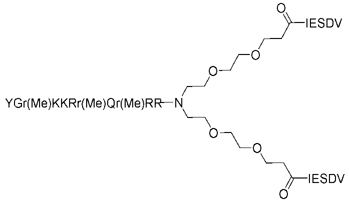 | H58 | 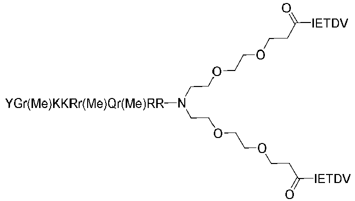 |
| H59 | 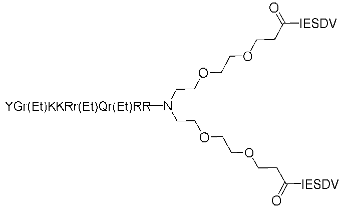 | H60 | 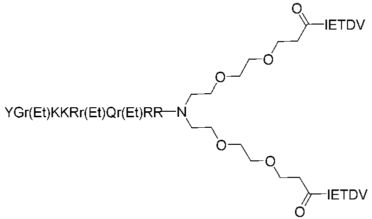 |
| H61 | 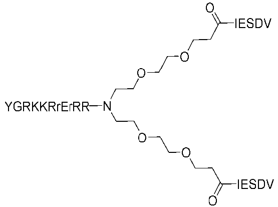 | H62 | 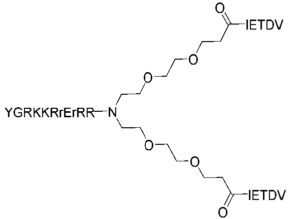 |
| H63 | 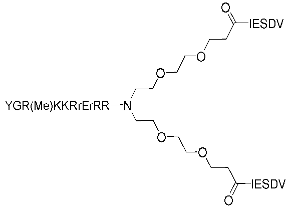 | H64 | 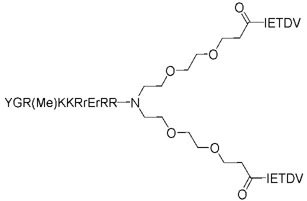 |
| H65 | 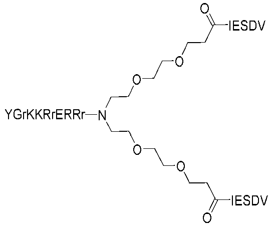 | H66 | 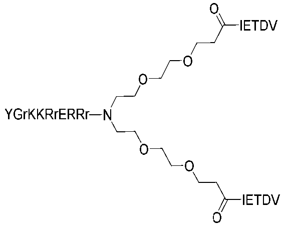 |
| H67 | 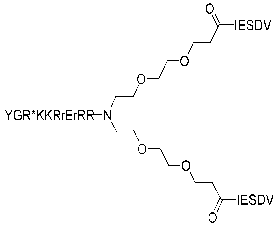 | H68 | 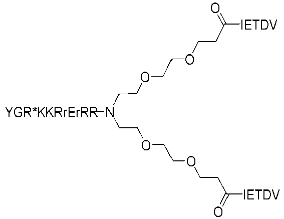 |
| H69 | 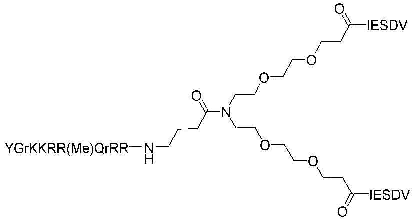 | H70 | 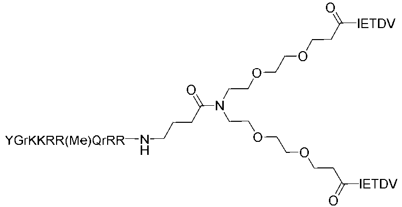 |
| H71 | 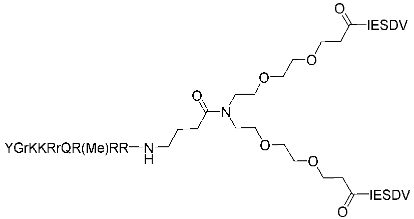 | H72 | 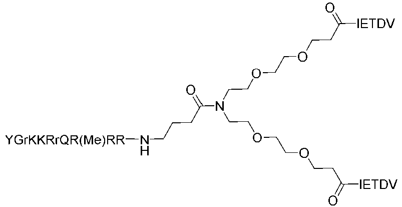 |
| H73 | 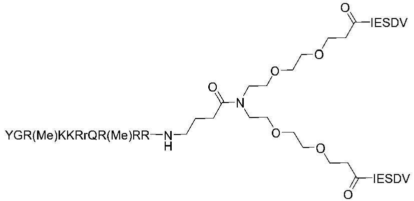 | H74 | 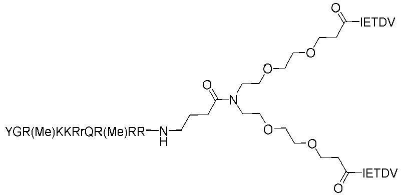 |
| H75 | 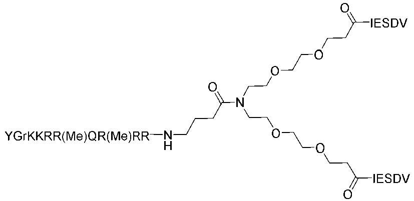 | H76 | 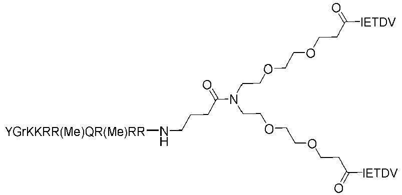 |
| H77 | 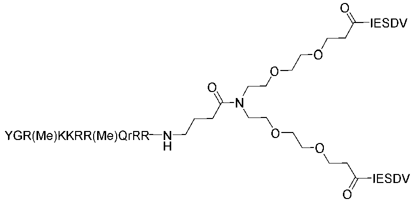 | H78 | 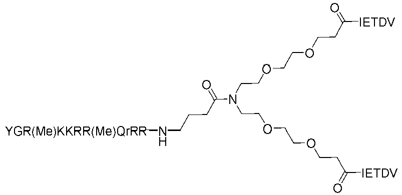 |
| H79 | 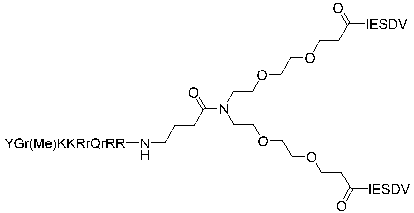 | H80 | 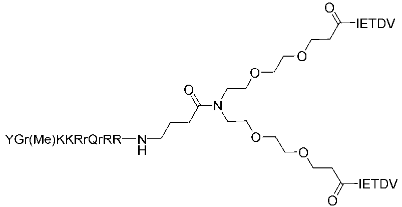 |
| H81 | 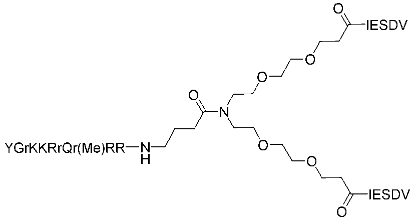 | H82 | 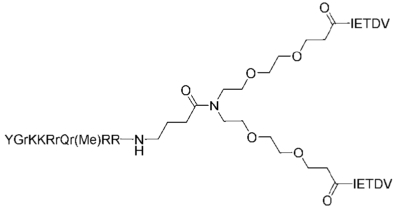 |
| H83 | 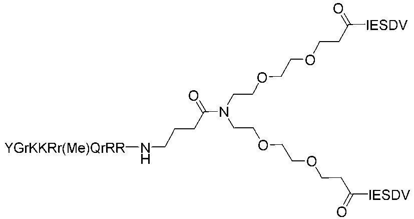 | H84 | 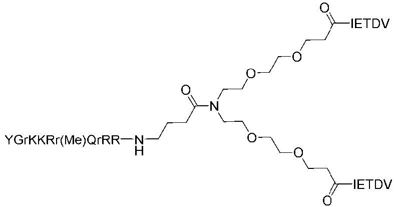 |
| H85 | 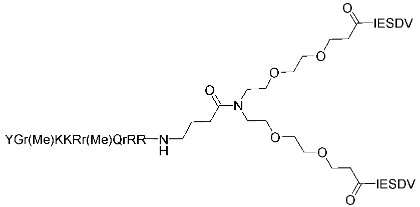 | H86 | 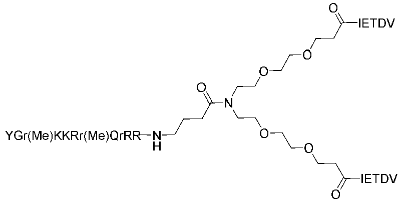 |
| H87 | 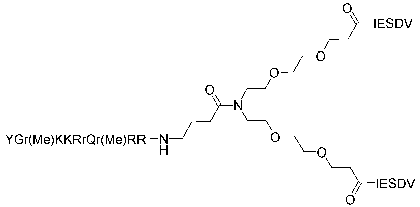 | H88 | 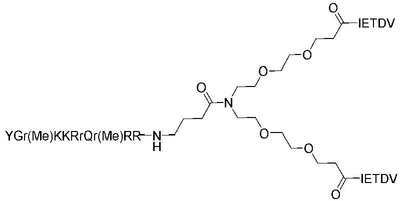 |
| H89 | 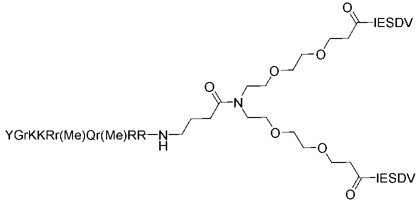 | H90 | 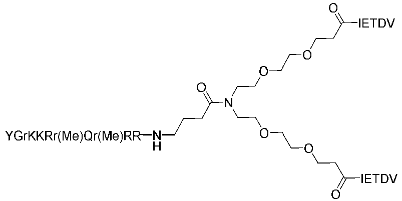 |
| H91 | 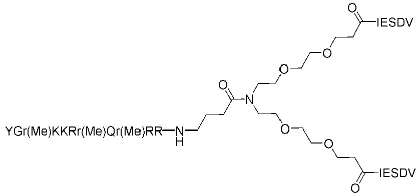 | H92 | 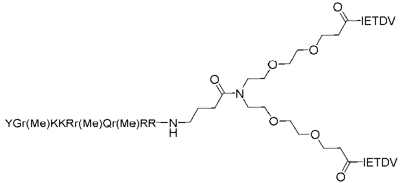 |
| H93 | 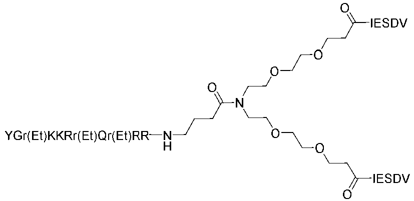 | H94 | 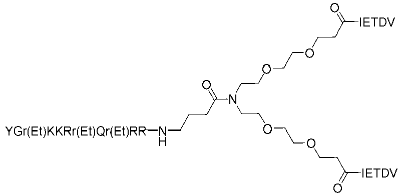 |
| H95 | 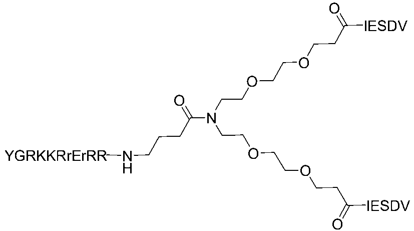 | H96 | 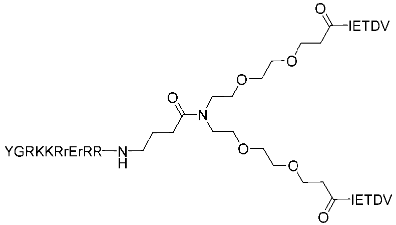 |
| H97 | 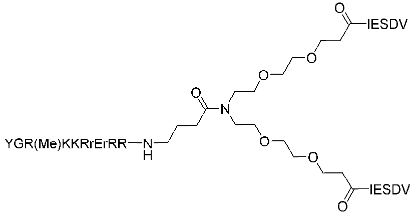 | H98 | 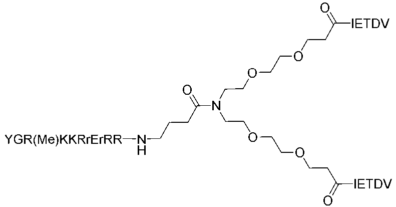 |
| H99 | 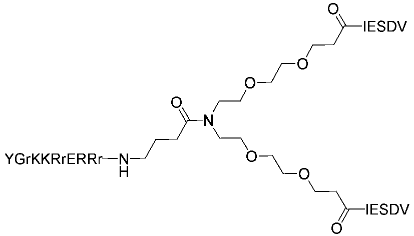 | H100 | 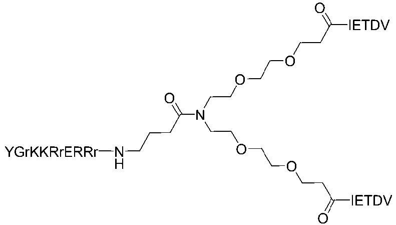 |
| H101 | 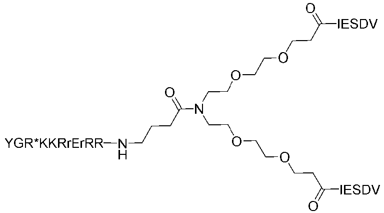 | H102 | 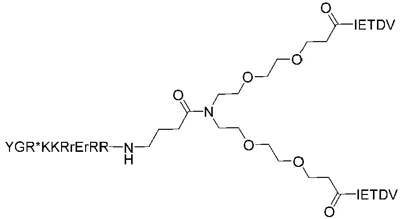 |

**Table I**

| No. | Compound | No. | Compound |
|---|---|---|---|
| I1 | YGrKKRrER(Me)RRkLSSIESDV | I2 | YGrKKRrER(Me)RRkLSSIETDV |
| I3 | YGrKKRrQR(Me)RRKLSSIESDV | I4 | YGrKKRrQR(Me)RRKLSSIETDV |
| I5 | YGrKKRrQR(Me)RRK(Me)LSSIESDV | I6 | YGrKKRrQR(Me)RRK(Me)LSSIETDV |
| I7 | YGrKKRrQR(Me)RR(Me)KLSSIESDV | I8 | YGrKKRrQR(Me)RR(Me)KLSSIETDV |
| I9 | YGrKKRrQR(Me)RR(Me)K(Me)LSSIE SDV | I10 | YGrKKRrQR(Me)RR(Me)K(Me)LSSIE TDV |
| I11 | YGrKKRrQRRRkLSSIESDV | I12 | YGrKKRrQRRRkLSSIETDV |
| I13 | YGR*KKRrQR(Me)RRkLSSIESDV | I14 | YGR*KKRrQR(Me)RRkLSSIETDV |
| I15 | YGR*KKRrER(Me)RRkLSSIESDV | I16 | YGR*KKRrER(Me)RRkLSSIESDV |
| I17 | 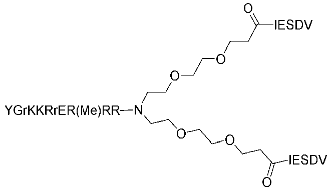 | I18 | 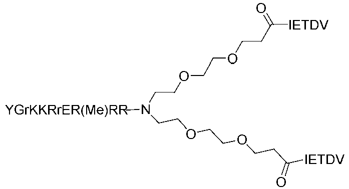 |
| I19 | 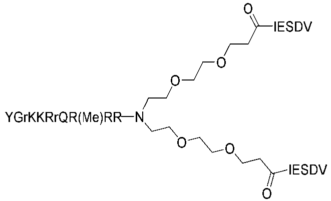 | I20 | 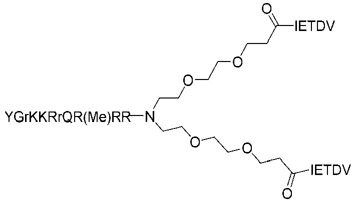 |
| I21 | 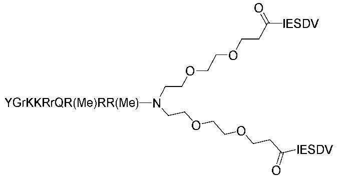 | I22 | 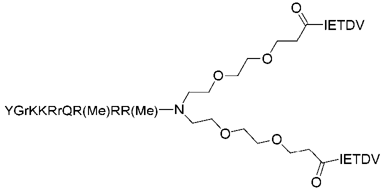 |
| I23 | 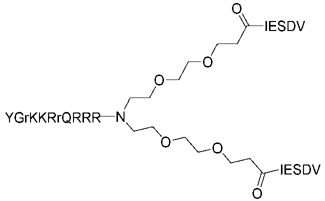 | I24 | 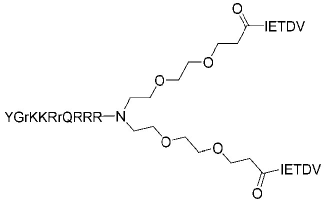 |
| I25 | 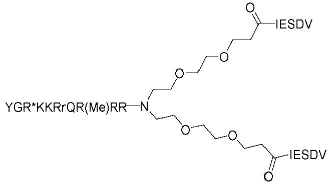 | I26 | 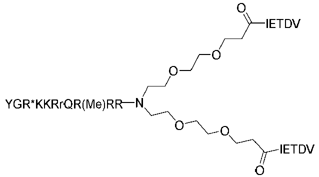 |
| I27 | 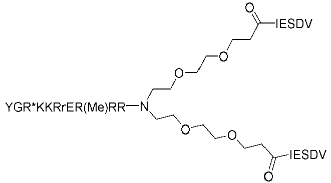 | I28 | 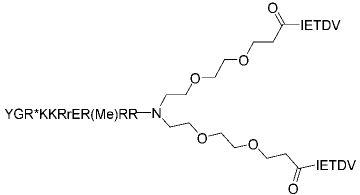 |

**Table J**

| No. | Compound | No. | Compound |
|---|---|---|---|
| 1 | YGrKKRrQrRkLSSIESDV | 2 | YGrKKRrQrRkLSSIETDV |
| 3 | YGrKKRrQrRkLSSIETAV | 4 | YGrKKRrQrRkLSSKETLV |
| 5 | YGrKKRrQrRkLSSKETTV | 6 | YGrKKRrQrRkLSSISTDV |
| 7 | YGrKKRrQrRkLSSVETVV | 8 | YGrKKRrErRkLSSIESDV |
| 9 | YGrKKRrErRkLSSIETDV | 10 | YGrKKRrErRkLSSIETAV |
| 11 | YGrKKRrErRkLSSKETLV | 12 | YGrKKRrErRkLSSKETTV |
| 13 | YGrKKRrErRkLSSISTDV | 14 | YGrKKRrErRkLSSVETVV |
| 15 | YGrKKRrErRRkLSSIESDV | 16 | YGrKKRrErRRkLSSIETDV |
| 17 | YGrKKRrErRRKLSSIETAV | 18 | YGrKKRrErRRkLSSKETLV |
| 19 | YGrKKRrErRRkLSSKETTV | 20 | YGrKKRrErRRkLSSISTDV |
| 21 | YGrKKRrErRRkLSSVETVV | 22 | YGrKKRrQrRKLSSIESDV |
| 23 | YGrKKRrQrRKLSSIETDV | 24 | YGrKKRrQrRKLSSIETAV |
| 25 | YGrKKRrQrRKLSSKETLV | 26 | YGrKKRrQrRKLSSKETTV |
| 27 | YGrKKRrQrRKLSSISTDV | 28 | YGrKKRrQrRKLSSVETVV |
| 29 | YGrKKRrErRKLSSIESDV | 30 | YGrKKRrErRKLSSIETDV |
| 31 | YGrKKRrErRKLSSIETAV | 32 | YGrKKRrErRKLSSKETTV |
| 33 | YGrKKRrErRKLSSISTDV | 34 | YGrKKRrErRKLSSVETVV |
| 35 | YGrKKRrErRKLSSKETTV | 36 | YGrKKRrErRRKLSSIESDV |
| 37 | YGrKKRrErRRKLSSIETDV | 38 | YGrKKRrErRRKLSSIETAV |
| 39 | YGrKKRrErRRKLSSKETLV | 40 | YGrKKRrErRRKLSSKETTV |
| 41 | YGrKKRrErRRKLSSISTDV | 42 | YGrKKRrErRRKLSSVETVV |
| 43 | 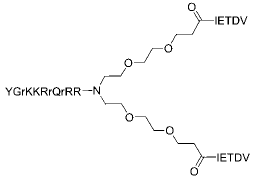 | 44 | 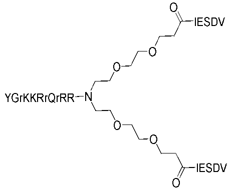 |
| 45 | 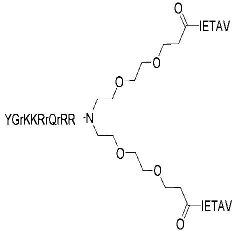 | 46 | 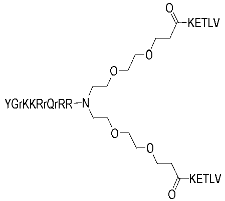 |
| 47 | 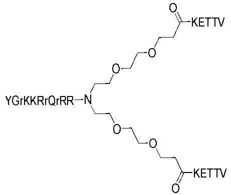 | 48 | 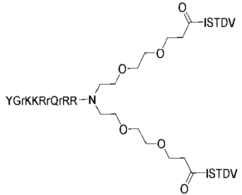 |
| 49 | 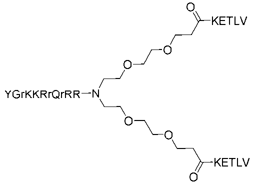 | *50* | 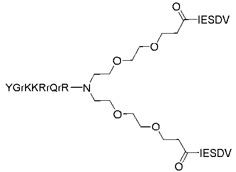 |
| 51 | 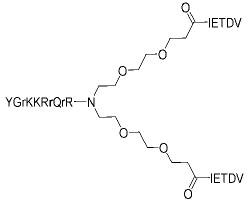 | 52 | 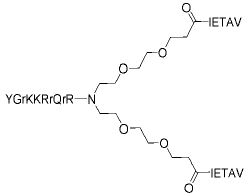 |
| 53 | 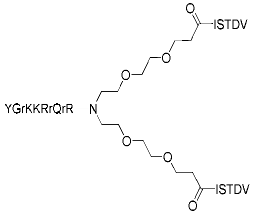 | 54 | 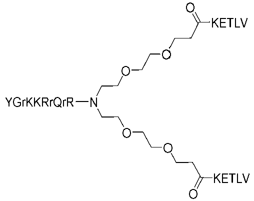 |
| 55 | 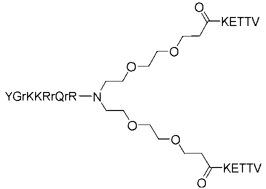 | 56 | 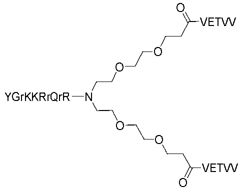 |
| 57 | 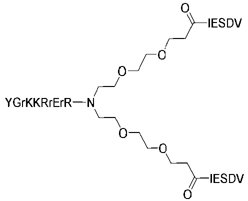 | 58 | 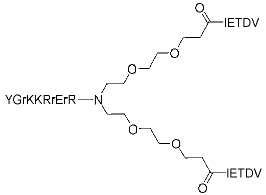 |
| 59 | 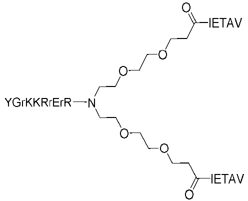 | 60 | 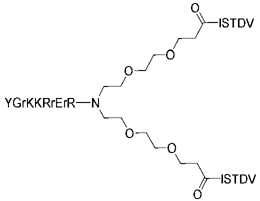 |
| 61 | 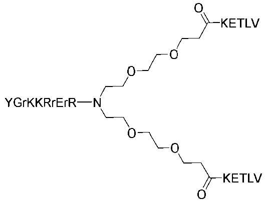 | 62 | 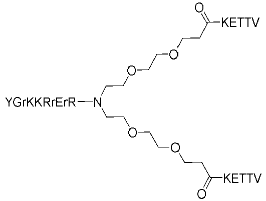 |
| 63 | 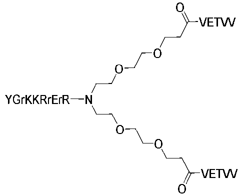 | 64 | 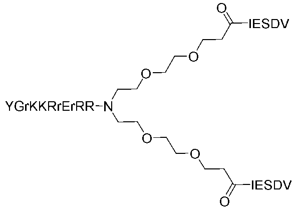 |
| 65 | 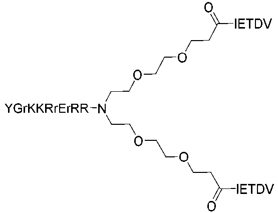 | 66 | 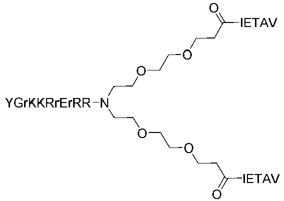 |
| 67 | 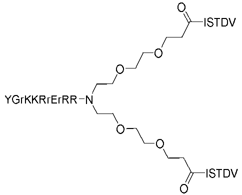 | 68 | 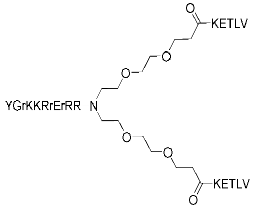 |
| 69 | 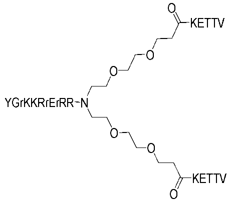 | 70 | 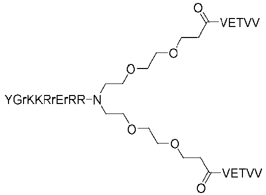 |
| 71 | 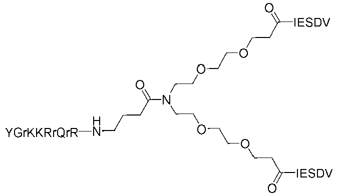 | 72 | 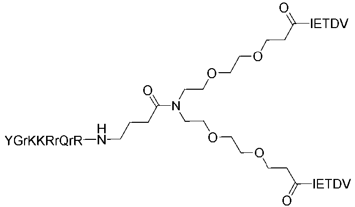 |
| 73 | 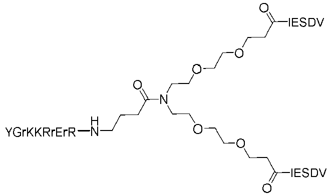 | 74 | 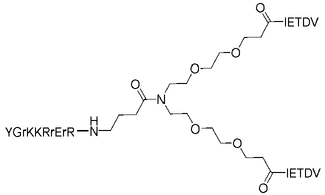 |
| 75 | 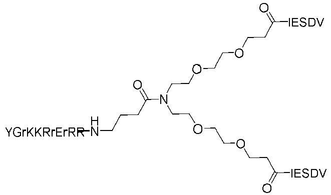 | 76 | 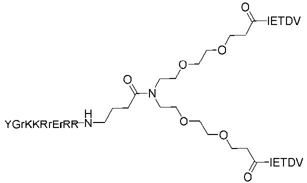 |
| 77 | 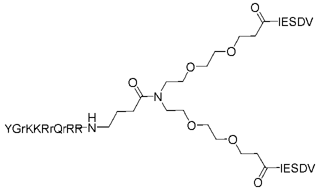 | 78 | 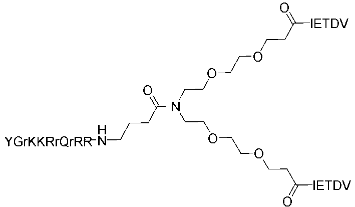 |
| 79 | 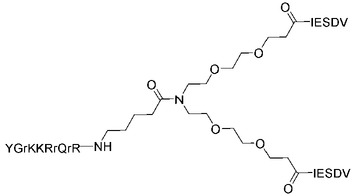 | 80 | 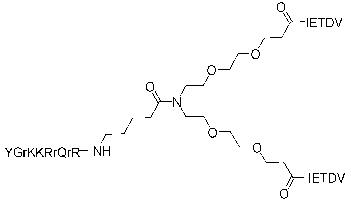 |
| 81 | 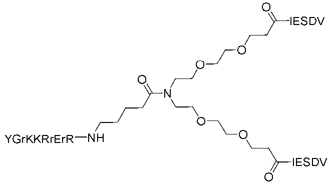 | 82 | 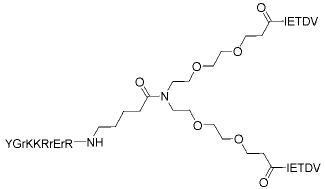 |
| 83 | 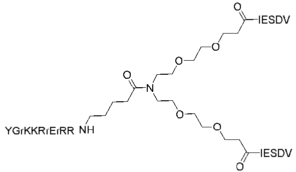 | 84 | 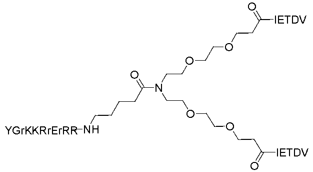 |
| 85 | 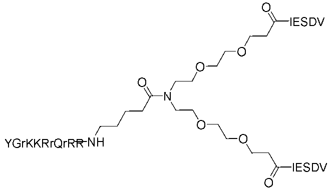 | 86 | 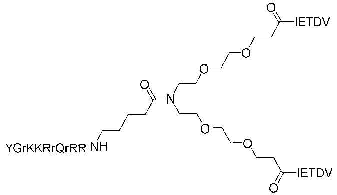 |
| 87 | 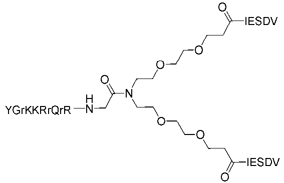 | 88 | 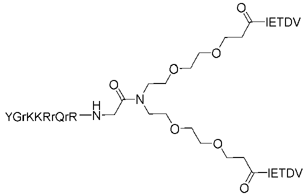 |
| 89 | 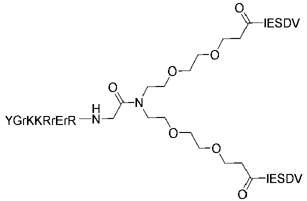 | 90 | 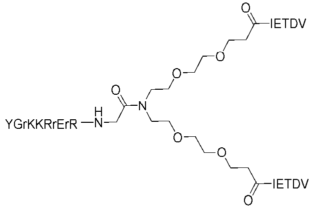 |
| 91 | 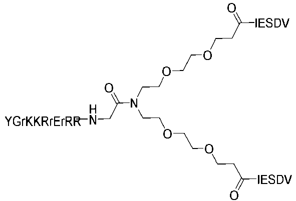 | 92 | 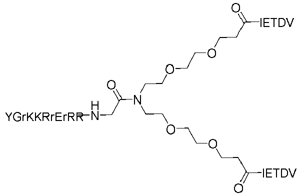 |
| 93 | 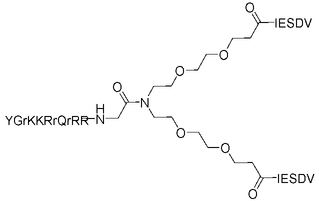 | 94 | 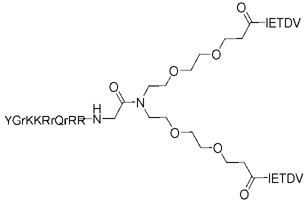 |
| 95 | 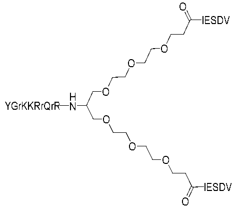 | 96 | 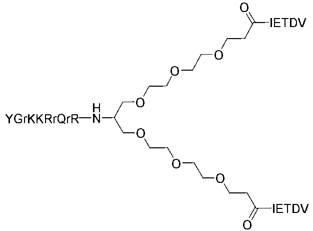 |
| 97 | 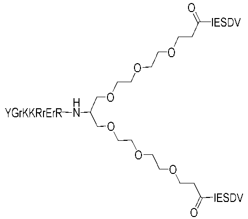 | 98 | 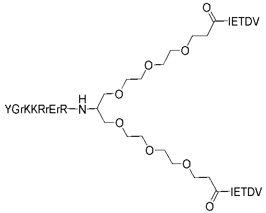 |
| 99 | 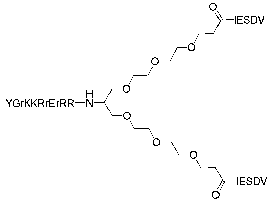 | 100 | 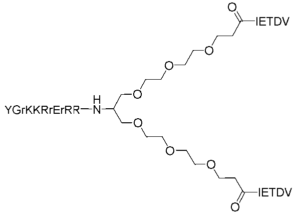 |
| 101 | 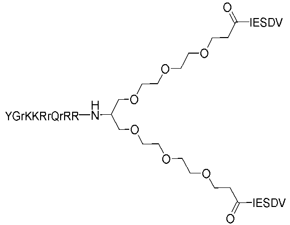 | 102 | 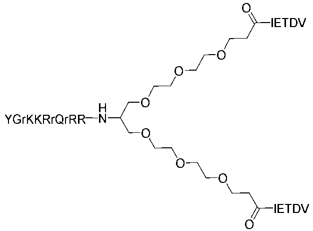 |
| 103 | 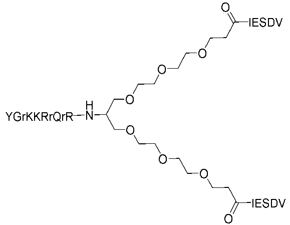 | 104 | 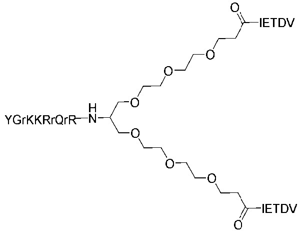 |
| 105 | 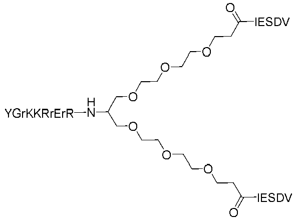 | 106 | 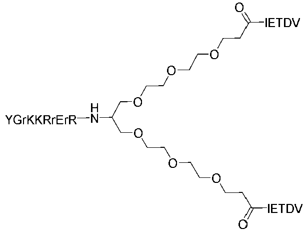 |
| 107 | 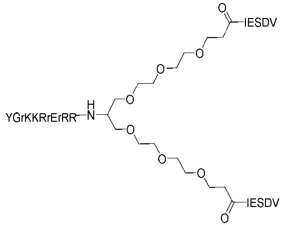 | 108 | 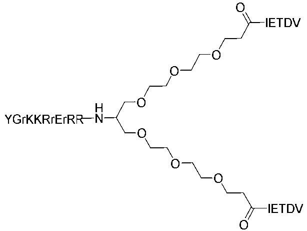 |
| 109 | 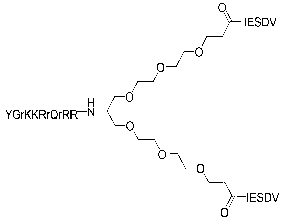 | 110 | 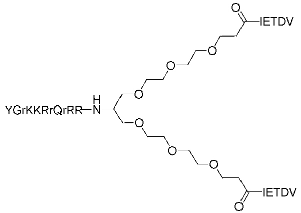 |
| 111 | 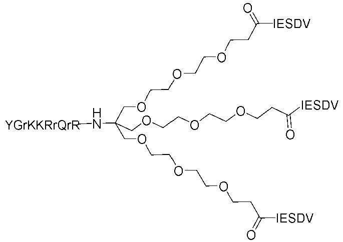 | 112 | 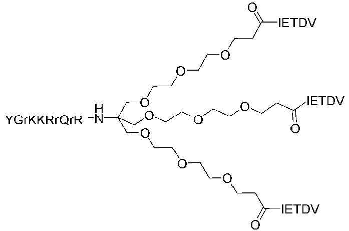 |
| 113 | 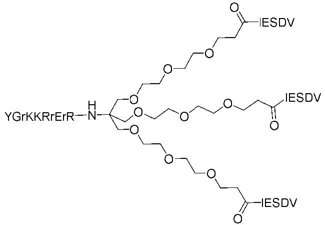 | 114 | 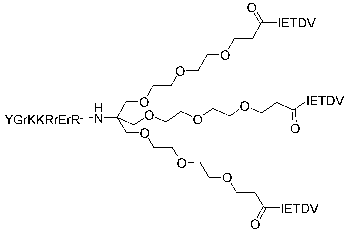 |
| 115 | 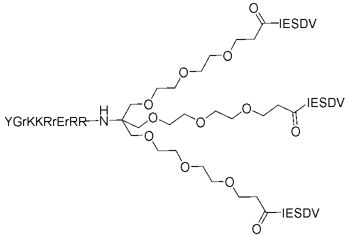 | 116 | 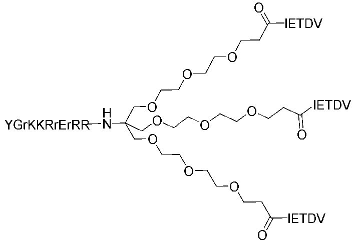 |
| 117 | 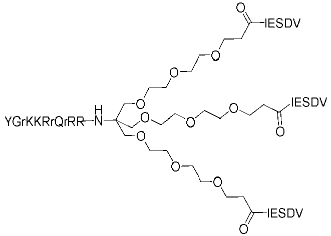 | 118 | 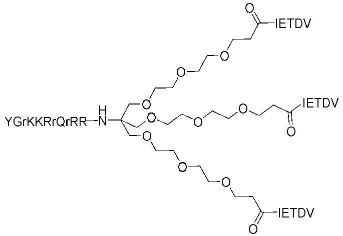 |
| 119 | 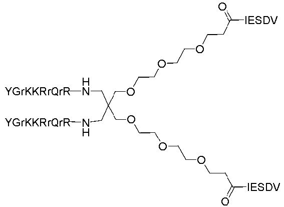 | 120 | 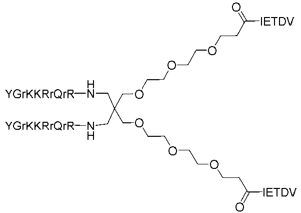 |
| 121 | 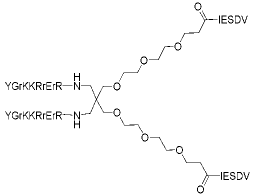 | 122 | 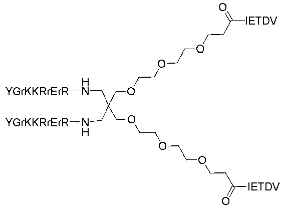 |
| 123 | 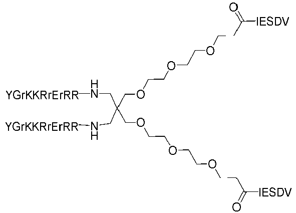 | 124 | 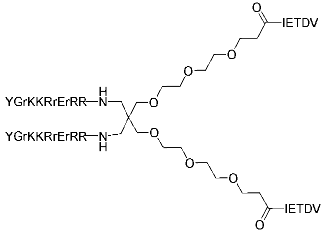 |
| 125 | 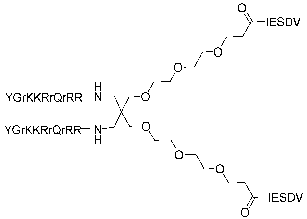 | 126 | 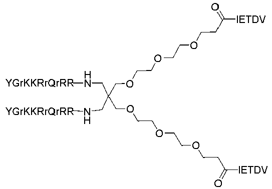 |
| 127 | 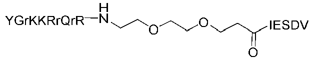 | 128 | 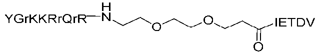 |
| 129 | 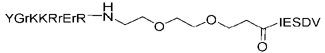 | 130 | 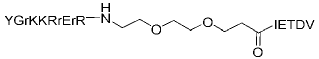 |
| 131 | 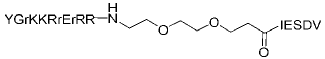 | 132 | 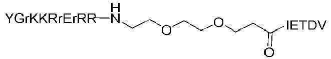 |
| 133 | 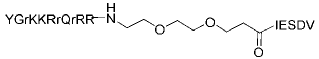 | 134 | 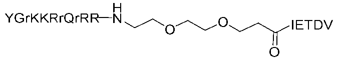 |

The present invention further provides a pharmaceutical composition, characterized in that the pharmaceutical composition comprises the PSD-95 domain inhibitor shown in formula (I) and one or more pharmaceutically acceptable carriers.

Another object of the present invention is to provide use of the PSD-95 domain inhibitor shown in formula (I) in the preparation of a medicament for the treatment of PSD-95 related diseases.

Another object of the present invention is to provide a cell-penetrating peptide, characterized in that the cell-penetrating peptide is selected from the group consisting of:
YGRKKRrQrR, YGrKKRRQrR, YGrKKRrQRR, YGRKKRrErRR, YGrKKRrERRr, YGrKKRrErRR, YGRKKRrErR, YGrKKRrERr, YGrKKRrErR, YGR^{#}KKRrQrRR, YGR^{#}KKRR^{#}QrRR, YGR^{#}KKRrQR^{#}RR, YGR^{#}KKRR*QR^{#}RR, YGrKKRR^{#}QrRR, YGrKKRR^{#}QR^{#}RR and YGrKKRrQR^{#}RR, wherein an uppercase letter denotes an L-amino acid, lowercase letter denotes a D-amino acid, and the symbol # denotes C₁-C₆ alkyl modification, C₂-C₆ acyl modification or a halogen modification;
Preferably, the C₁-C₆ alkyl modification is selected from the group consisting of methyl and ethyl; the C₂-C₆ acyl is acetyl;
More referably, the cell-penetrating peptide is selected from the group consisting of: YGrKKRrQrR, YGrKKRrErRR, YGrKKRrQR(Me)RR, YGrKKRrER(Me)RR, YGR*KKRrQR(Me)RR, and YGR*KKRrER(Me)RR, wherein, the

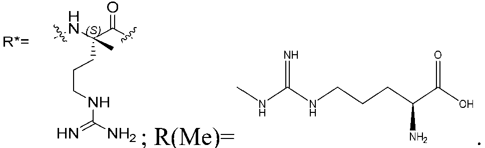

Further, as a preferred technical solution of the present invention, the cell-penetrating peptide is selected from the group consisting of the following compounds:

| |
|---|
| YGR*KKRRQRRR, YGR*KKRrQRRR, YGR*KKRRQrRR, YGR*KKRrQrRR |
| YGRKKRR*QRRR, YGrKKRR*QRRR, YGrKKRR*QrRR, YGRKKRR*QrRR |
| YGRKKRRQR*RR, YGrKKRRQR*RR, YGrKKRrQR*RR, YGrKKRrQR*RR |
| YGrKKRrQrR, YGR*KKRrQR(Me)RR, YGR*KKRrER(Me)RR |
| YGR(Me)KKrQrRR, YGrKKRR(Me)QrRR, YGrKKRrQR(Me)RR |
| YGR(Me)KKRrQR(Me)RR, YGrKKRR(Me)QR(Me)RR, YGR(Me)KKRR(Me)QrRR |
| YGr(Me)KKRrQrRR, YGrKKRrQr(Me)RR, YGrKKRr(Me)QrRR |
| YGr(Me)KKRr(Me)QrRR, YGr(Me)KKRrQr(Me)RR, YGrKKRr(Me)Qr(Me)RR |
| YGr(Me)KKRr(Me)Qr(Me)RR, YGR(Et)KKRR(Et)QR(Et)RR |
| YGRKKRrErRR, YGrKKRrERRr, YGrKKRrErRR |
| YGRKKRrErR, YGrKKRrERr, YGrKKRrErR, YGrKKRrQrR |
| YGrKKRrER(Me)RR, YGrKKRrQR(Me)RR(Me) |
| YGR(Me)KKRR(Me)QR(Me)RR, YGR(Me)₂KKRR(Me)₂QR(Me)₂RR |
| YGR(Ac)KKRR(Ac)QR(Ac)RR, YGR(Ac)₂KKRR(Ac)₂QR(Ac)₂RR, YGR(Ac)KKRrQrRR, |

wherein,

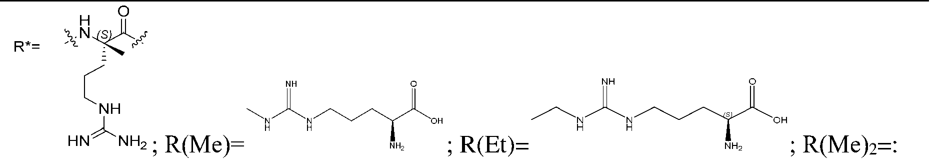

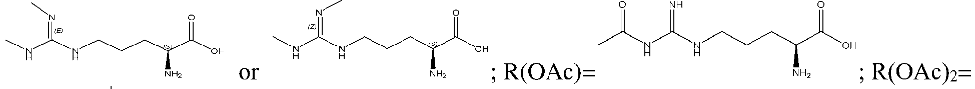

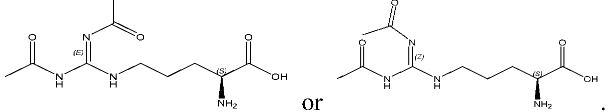

Further, the present invention also provides a plasmin-resistance peptide, characterized in that the plasmin-resistance peptide comprises a cell-penetrating peptide according to the present invention, and the cell-penetrating peptide is connected to a functional peptide of the plasmin-resistance peptide directly or via a linker; wherein the functional peptide is selected from the group consisting of one or more of IETDV, IETAV, IESDV, KETLV, KETTV, ISTDV and VETVV.

Further, as a preferred technical solution of the present invention, the linker is selected from the group consisting of KLSS, kLSS, 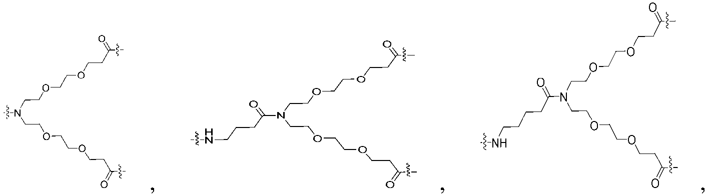 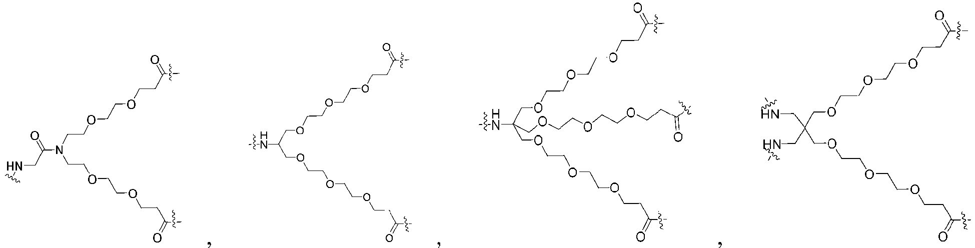 and

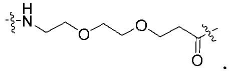

Further, as a preferred technical solution of the present invention, provided is use of the cell-penetrating peptide for delivering a plasmin-resistance peptide.

Further, the present invention also provides a method of preparing a PSD-95 domain inhibitor shown in formula (I):
The compounds of the present invention may be synthesized with reference to a conventional synthetic method, for example, the method disclosed in Patent CN111560050A, or may be synthesized using the following method:
1) Synthesis of the linker Ns-N[(PEG)2-COOH]2: Loading Fmoc-NH-PEG2-CH₂CH₂COOH onto the resin, then removing the Fmoc group using DMF containing 20% piperidine; slowly adding a DCM solution containing *o*-nitrobenzenesulfonyl chloride (NsCl) and stirring the mixture for reaction; subsequently, treating the resin with THF containing triphenylphosphine and THF containing HO-PEG2-CH₂CH₂COOtBu; adding diisopropyl azodicarboxylate dropwise and shaking the mixture for reaction; thoroughly rinsing the resin with THF and DCM, then drying under vacuum. Treating the resin with a cocktail of TFA/triisopropylsilane/water, filtering the resin, then rinsing the resin with TFA and DCM, and combining and concentrating the rinsate and filtrate. Dissolving the concentrated residue in water/acetonitrile and lyophilizing to obtain Ns-N[(PEG)2-COOH]2, and the linker is used directly for subsequent synthesis.
2) Synthesis of Ile-Glu(OtBu)-Thr(tBu)-Asp(OtBu)-Val-Wang Resin: synthesizing the resin-containing peptide sequence Ile-Glu(OtBu)-Thr(tBu)-Asp(OtBu)-Val-Wang Resin (IETDV) in anhydrous DMF solvent using pre-loaded Fmoc-Val-Wang-resin in the presence of a coupling agent HBTU/DIPEA.
3) Synthesis of NPEG(4)-based dimer: Activateing the Ns-N[(PEG)2-COOH]2 linker using HBTU and DIPEA, adding into DMF containing Ile-Glu(OtBu)-Thr(tBu)-Asp(OtBu)-Val-Wang Resin, and shaking the mixture for reaction. Adding DMF containing DBU, then adding DMF containing mercaptoethanol, and shaking the mixture to remove the Ns protecting group. Draining the resin, rinsing with DMF, and repeating the treatment once using mercaptoethanol/DBU. Successively rinsing the resin using DMF, DCM, MeOH and DCM to obtain the NPEG(4)-based dimer: 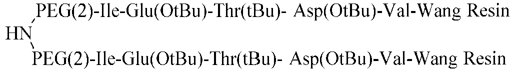
4) Synthesis of PSD-95 domain inhibitor: synthesizing a resin-containing peptide sequence using the NPEG(4)-based dimer with HBTU/DIPEA as coupling agent and anhydrous DMF as solvent:
   Fmoc-Tyr(tBu)-Gly-Arg(Me,Pbf)-Lys(Boc)-Lys(Boc)-Arg(Pbf)-Arg(Me,Pbf)-Gln(Trt)-Arg(Me,Pbf)-Arg(Pbf)-Arg(Pbf)-{N,N-Di(PEG2-Ile-Glu(OtBu)-Thr(tBu)-Asp(OtBu)-Val-Wang Resin)}. Removing the Fmoc protecting group using a DMF solution containing 20% piperidine. Adding a cleavage reagent to the resin, stirring well, and stirring for the reaction at room temperature; filtering and collecting the filtrate; washing the resin again, combining the filtrates and concentrating the combined filtrates under reduced pressure; adding anhydrous ether to precipitate the product, and drying the precipitate under reduced pressure to obtain a crude compound; purifying the crude compound using high performance liquid chromatography.

For clarity, general terms used in the description of compounds are defined herein.

Unless otherwise stated, the following terms and phrases used herein are intended to have the meanings set forth below. A specific term or phrase should not be considered indefinite or unclear without a special definition, but should be understood according to its ordinary meaning. When a trade name is mentioned herein, it is intended to refer to the corresponding commercial product or its active ingredient. The term "pharmaceutically acceptable" as used herein is respect to those compounds, materials, compositions and/or dosage forms which, within the scope of sound medical judgment, are suitable for use in contact with human and animal tissues without excessive toxicity, irritation, allergic reaction, or other problems or complications commensurate with a reasonable benefit/risk ratio.

The term TAT is a cell-penetrating peptide, and the TAT is selected from the group consisting of: YGR(Me)KKRR(Me)QR(Me)RR, YGR(Me)₂KKRR(Me)₂QR(Me)₂RR, YGR(Me)KKRrQrRR, YGR(Ac)KKRR(Ac)QR(Ac)RR, YGR(Ac)₂KKRR(Ac)₂QR(Ac)₂RR and YGR(Ac)KKRrQrRR; Me is methyl, and Ac is acetyl,
the structural formula of R(Me) is: 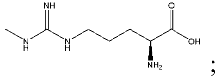 the structural formula of R(Et) is: 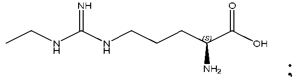 the structural formula of R(Me)₂ is: 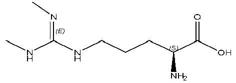 or 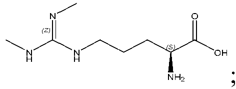 the structural formula of R(OAc) is: 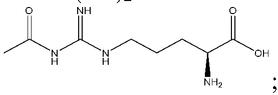 a structural formula of R(OAc)₂ is: 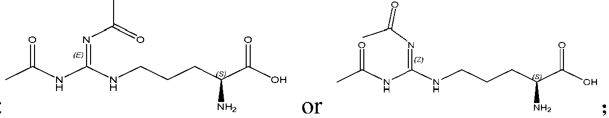 in the TAT, an uppercase letter denotes an L-amino acid, and a lowercase letter denotes a D-amino acid.

The term "alkyl" refers to a saturated aliphatic hydrocarbon group, including linear and branched chain group having 1 to 20 carbon atoms. Preferably, the alkyl is an alkyl containing 1 to 6 carbon atoms. Nonlimiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, and various branched isomers thereof.

The term "acyl" refers to a group obtained by replacing the H atom of aldehyde group with a C₁-C₅ alkyl, for example, acetyl, propionyl, butyryl and the like.

The term "R^{#}" refers to an arginine modified by C₁-C₆ alkyl, C₂-C₆ acyl or halogen, and the modifying group may be 1, 2 or more.

The term "pharmaceutically acceptable salt" refers to a salt of compounds of the present invention, prepared from a compound having a specific substituent found in the present invention and a pharmaceutically acceptable acid or base.

The term "pharmaceutically acceptable carrier" refers to any formulation carrier or medium that is capable of delivering an effective amount of the active substance of the present invention, does not interfere with the biological activity of the active substance, and has no toxicity or side effect on the host or patient. Representative carriers include water, oils, vegetables and minerals, cream bases, lotion bases, ointment bases, and the like. These bases include suspending agent, viscosity-increasing agent, transdermal promotor and the like. Their formulations are well known to technicans in the field of cosmetics or in the field of topical drugs. For further information regarding carriers, reference can be found in Remington: The Science and Practice of Pharmacy, 21st Ed. in Lippincott, Williams & Wilkins (2005), the content of which is incorporated herein by reference.

The term "excipient" generally refers to the carrier, diluent and/or medium required to formulate an effective pharmaceutical composition.

For a drug or pharmacologically active agent, the term "effective amount" or "therapeutically effective amount" refers to a sufficient amount of a drug or agent that is non-toxic but achieves the desired effect. For the oral dosage form of the present invention, the "effective amount" of one active substance in the composition refers to the amount required to achieve the desired effect when used in combination with another active substance in the composition. The determination of an effective amount varies from person to person, depending on the age and general condition of the recipient, as well as the specific active substance, and the appropriate effective amount in individual cases may be determined by those skilled in the art on the basis of routine experiments.

The term "active ingredient", "therapeutic agent", "active substance" or "active agent" refers to a chemical entity that is effective in treating the target disorder, disease or condition.

"Optional" or "optionally" means that the event or circumstance subsequently described may, but does not necessarily, occur, and that the description includes instances where the event or circumstance occurs as well as instances where the event or circumstance does not occur.

The compounds of the present invention can be prepared by a variety of synthetic methods well-known to those skilled in the art, including the specific embodiments enumerated below, embodiments resulting from combinations thereof with other methods of chemical synthesis, and equivalent alternatives well-known to those skilled in the art. Preferred embodiments include, but are not limited to, the examples of the present invention.

### BRIEF DESCRIPTION OF THE DRAWINGS

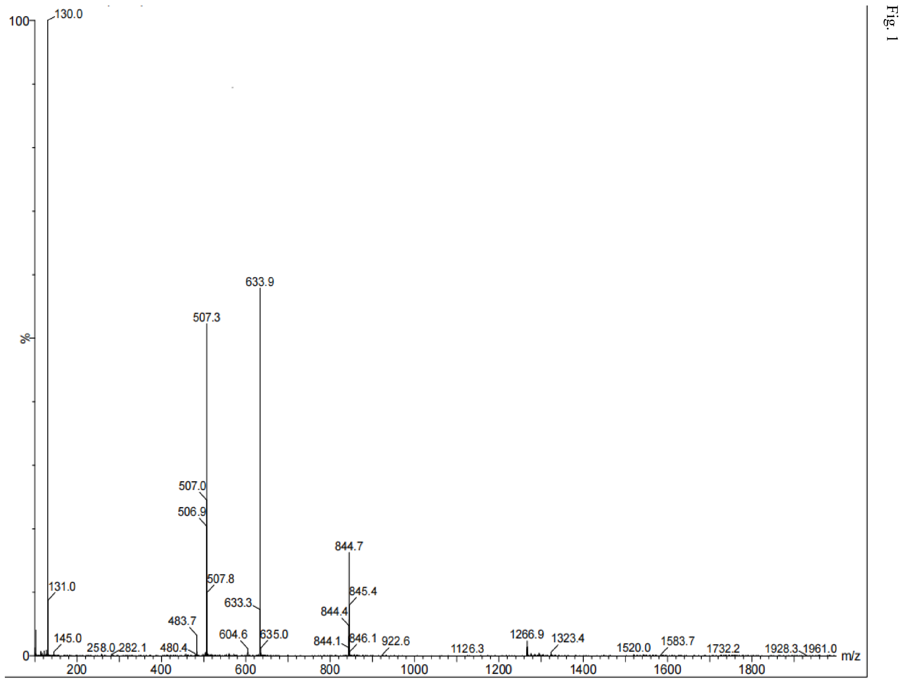

1. Fig. 1 shows the mass spectrum of compound **H3;**

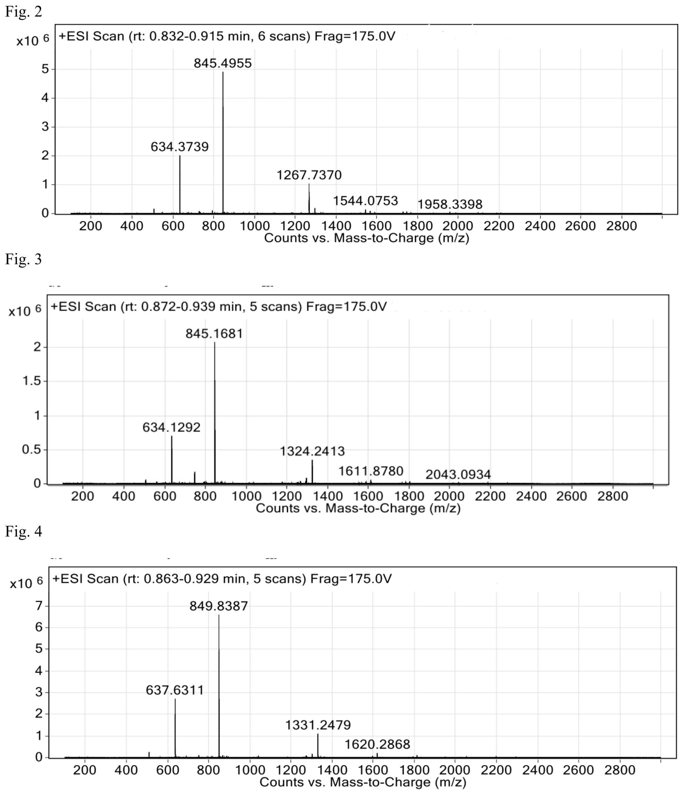

2. Fig. 2 shows the mass spectrum of compound **I1**;
3. Fig. 3 shows the mass spectrum of compound **G3;**
4. Fig. 4 shows the mass spectrum of compound **I13**;
5. Fig. 5 shows the mass spectrum of compound **I15**;
6. Fig. 6 shows the results of stability of compounds **I1**, **G3, I13** and **I15** in human plasma;

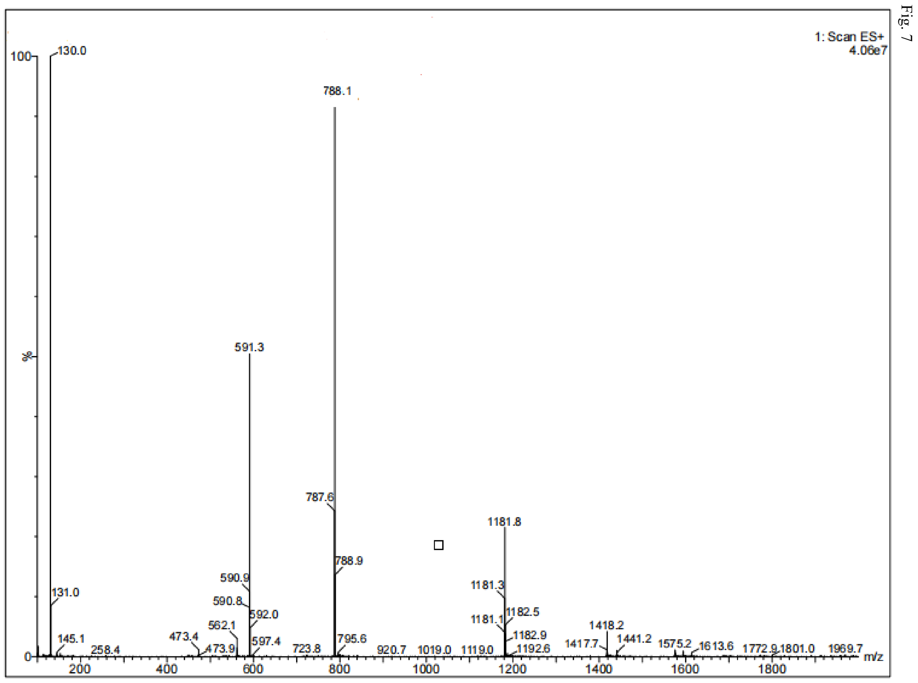

7. Fig. 7 shows the mass spectrum of compound **1;**

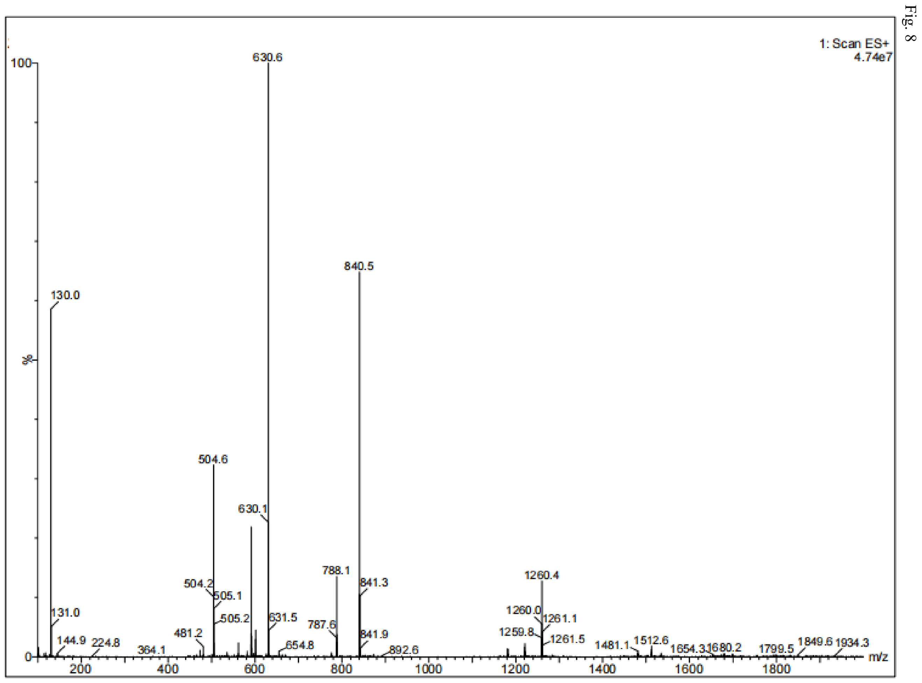

8. Fig. 8 shows the mass spectrum of compound **15;**

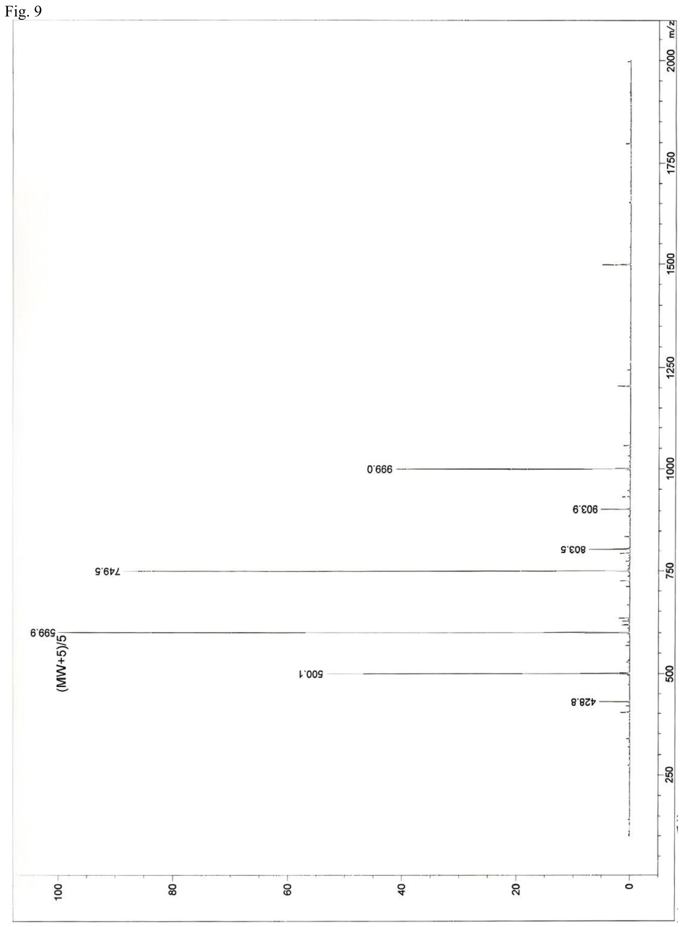

9. Fig. 9 shows the mass spectrum of compound **43;**

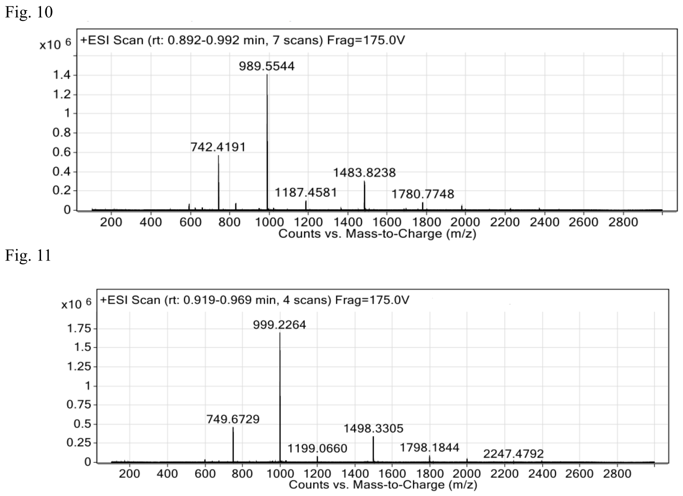

10. Fig. 10 shows the mass spectrum of compound **44;**
11. Fig. 11 shows the mass spectrum of compound **65;**

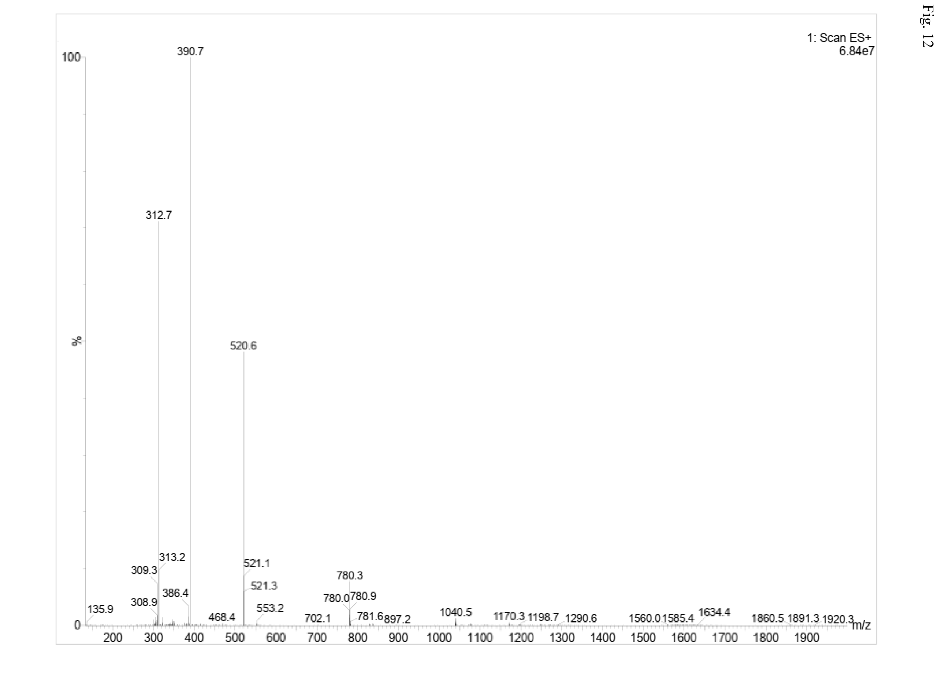

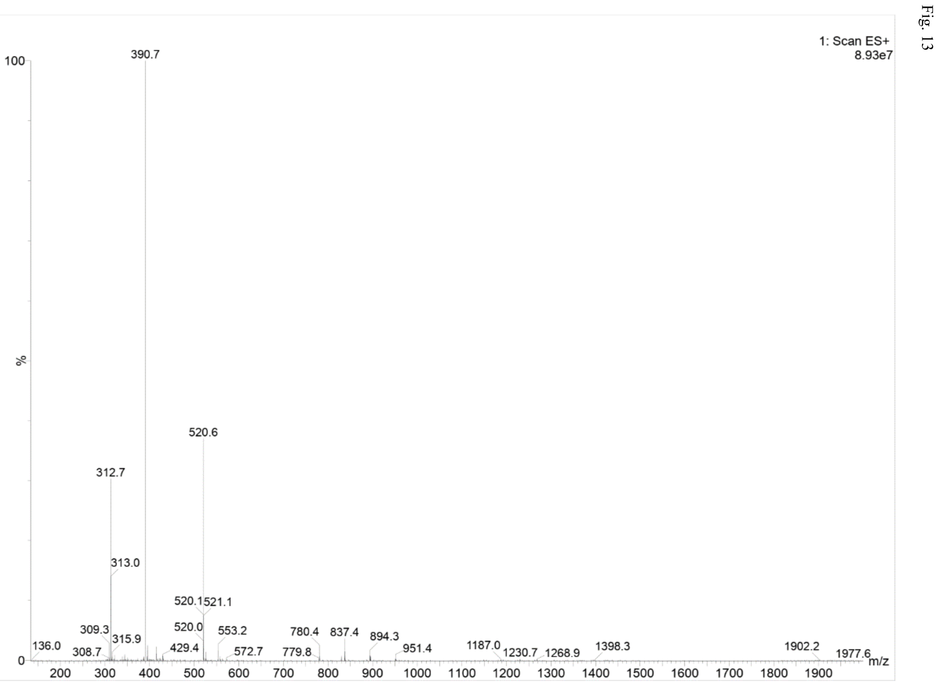

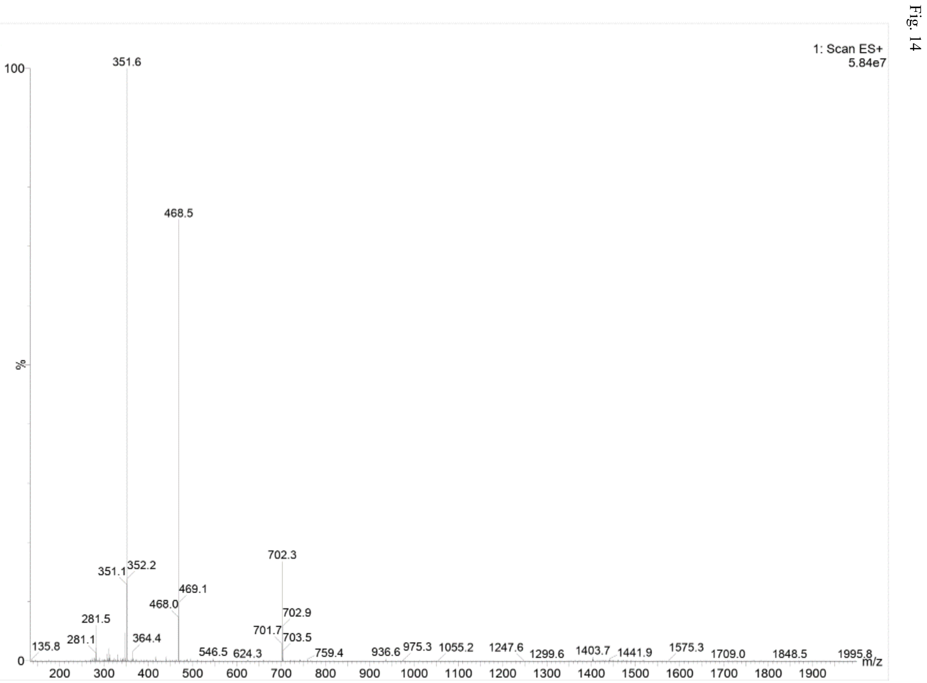

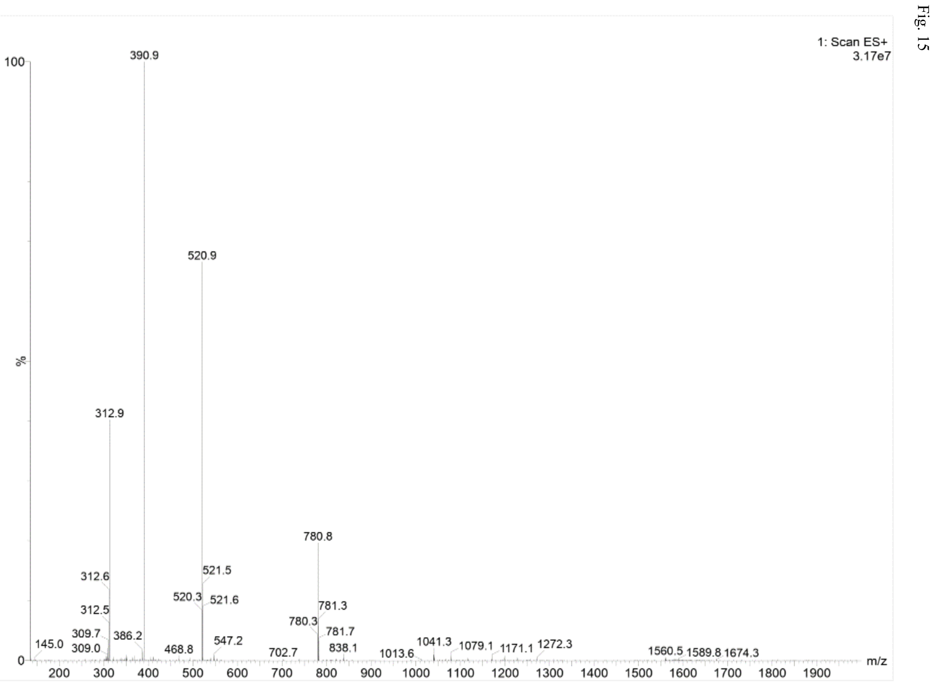

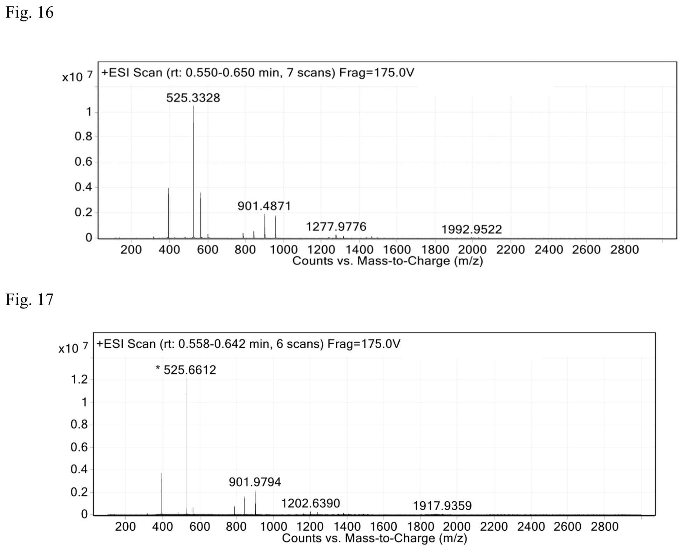

12. Fig. 12-Fig. 17 show mass spectra of the cell-penetrating peptides **TAT-1** to **TAT-6,** in which TAT-1 is the TAT of NA-1 and TAT-2 is the TAT of NoNo42.

### DETAILED DESCRIPTION OF THE EMBODIMENTS

The present application is described in further detail below in conjunction with examples, but the embodiments of the present application are not limited thereto.

The structures of the compounds are determined by mass spectrometry (MS).
MS measurements are performed using Agilent 6120;
High performance liquid chromatography (HPLC) analysis is performed using an Agilent 1220 high performance liquid chromatography.

In the specific embodiments of the present invention, the chemical names corresponding to the abbreviations used in the application documents are shown in Table 1.

**Table 1 List of chemical names and abbreviations**

| Single-Letter Abbreviati on | Abbreviati on | Chemical Name | Single-Letter Abbreviati on | Abbreviati on | Chemical Name |
|---|---|---|---|---|---|
| | Fmoc | 9-Fluorenylmethoxycarbo nyl | | OtBu | tert-butoxy |
| | tBu | tert-butyl | | Boc | tert-Butyloxycarbonyl |
| | Trt | Triphenylmethyl | | Pbf | (2,3-dihydro-2,2,4,6,7-pentamethylbenzofur an-5-yl)sulfonyl |
| S | Ser | Serine | L | Leu | Leucine |
| E | Glu | Glutamic acid | R | Arg | Arginine |
| D | Asp | Aspartic acid | Q | Gln | Glutamine |
| Y | Tyr | Tyrosine | I | Ile | Isoleucine |
| G | Gly | Glycine | K | Lys | Lysine |
| V | Val | Valine | k | D-Lys | D-Lysine |
| r | D-Arg | D-Arginine | T | Thr | Threonine |

An uppercase letter denotes an L-amino acid, and a lowercase letter denotes a D-amino acid.

### Example 1

### Synthesis and Characterization of Compound A1

YGR(Me)KKRR(Me)QR(Me)RRkLSSIESDV **A1**

### 1) Synthesis and Characterization of Compound A1 Peptide Resin

**wherein, the structure of the compound A1 peptide resin is Boc-Tyr(tBu)-Gly-Arg(Me,Pbf)-Lys(Boc)-Lys(Boc)-Arg(Pbf)-Arg(Me,Pbf)-Gln(Trt)-Arg(Me,Pbf)-Arg(Pbf)-Arg(Pbf)-D-Lys(Boc)-Leu-Ser(tBu)-Ser(tBu)-Ile-Glu(OtBu)-Ser(tBu)-Asp(OtBu)-Val-Wang resin**
The Fmoc-Val-Wang Resin is used as the starting resin and sequentially coupled with the protected amino acids listed in Table 2 through the removal of the Fmoc protection and the coupling reaction, to prepare **Boc-Tyr(tBu)-Gly-Arg(Me,Pbf)-Lys(Boc)-Lys(Boc)-Arg(Pbf)-Arg(Me,Pbf)-Gln(Trt)-Arg(Me,Pbf)-Arg(Pbf)-Arg(Pbf)-D-Lys(Boc)-Leu-Ser(tBu)-Ser(tBu)-Ile-Glu(OtBu)-Ser(tBu)-Asp(OtBu)-Val-Wang** resin (for amino acids listed in Table 2, according to the structure of compound A1, uppercase letters denote the corresponding protected L-amino acids, and lowercase letters denote the corresponding protected D-amino acids).

**Table 2**

| Coupling Peptide Order n = | | Protected Amino Acid |
|---|---|---|
| | 2 | Fmoc-Asp(OtBu) |
| | 3 | Fmoc-Ser(tBu) |
| | 4 | Fmoc-Glu(OtBu) |
| | 5 | Fmoc-Ile |
| | 6 | Fmoc-Ser(tBu) |
| | 7 | Fmoc-Ser(tBu) |
| | 8 | Fmoc-Leu |
| | 9 | Fmoc-D-Lys(Boc) |
| | 10 | Fmoc-Arg(Pbf) |
| | 11 | Fmoc-Arg(Pbf) |
| | 12 | Fmoc-Arg(Me,Pbf) |
| | 13 | Fmoc-Gln(Trt) |
| | 14 | Fmoc-Arg(Me,Pbf) |
| | 15 | Fmoc-Arg(Pbf) |
| | 16 | Fmoc-Lys (Boc) |
| | 17 | Fmoc-Lys (Boc) |
| | 18 | Fmoc-Arg(Me,Pbf) |
| | 19 | Fmoc-Gly |
| | 20 | Boc-Tyr(tBu) |

wherein the structural formula of R(Me) is: 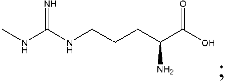 the structural formula of Fmoc-Arg(Me,Pbf) is:

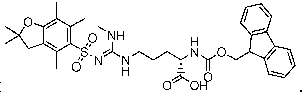

The specific steps are as follows:

### a) Coupling of the 2nd amino acid

0.03 mol of the 2nd protected amino acid and 0.03 mol HOBt were dissolved in an appropriate amount of DMF. Additionally, 0.03 mol DIC was slowly added to the solution of protected amino acid in DMF with stirring, and the mixture was stirred for reaction at room temperature for 30 minutes to obtain the activated protected amino acid solution, for future use.

0.01 mol of Fmoc-Val-Wang Resin (substitution value of ~0.5 mmol/g) was taken and deprotected using a 20% PIP/DMF solution for 25 minutes, followed by washing and filtering to obtain the Fmoc-deprotected resin.

The solution of the 2nd protected amino acid upon activated was added to the Fmoc-deprotected resin for the coupling reaction for 120-300 minutes. The resin was filtered and washed to obtain a resin containing 2 protected amino acids.

### b) Coupling of the 3rd to 20th amino acids

Using the same method described above, the aforementioned corresponding 3rd to 20th protected amino acids or fragments were sequentially coupled to obtain the compound A**1** peptide resin.

### 2) Preparation of Crude Compound A1

To the compound A1 peptide resin obtained in step 1) was added a cleavage cocktail with the volume ratio of TFA:Water: EDT = 95:5:5 (10 mL per gram of resin). The mixture was mixed well and stirred for reaction at room temperature for 3 hours. The reaction mixture was filtered by a sand funnel, and the filtrate was collected. The resin was washed three times with a small amount of TFA. The combined filtrates were concentrated under reduced pressure. Anhydrous ethyl ether was added to precipitate and the precipitate was then washed three times with anhydrous ethyl ether and dried under reduced pressure at 35-45°C to afford crude compound A1.

### 3) Preparation of Pure Compound A1

The crude compound A**1** obtained in step 2) was dissolved in 10% acetic acid solution, and the resulting solution was filtered through 0.45 µm mixed microporous filter membrane and purified for future use.

The purification was carried out using high performance liquid chromatography. The chromatographic packing material for purification was 10 µm reversed-phase C18, and two mobile phase systems were used for the purification alternately. The first mobile phase system was 0.1% TFA in water-0.1% TFA in acetonitrile, and the second mobile phase system was 50 mmol ammonium acetate in water-acetonitrile. The 77 mm*250 mm column flow rate was 90 mL/min. The elution was performed using a gradient system, and the sample was loaded cyclically for purification. The crude solution was loaded onto the column. The mobile phase was initiated for elution, and the main peak was collected. The obtained solution was evaporated to remove acetonitrile, and then filtered through a 0.45 µm membrane to obtain the concentrated solution of the purified compound A1 intermediate;

The salt exchange was carried out using high performance liquid chromatography. The mobile phase system was 1% acetic acid in water-acetonitrile, and the chromatographic packing material for purification was 10 µm reversed-phase C18. The 77 mm*250 mm column flow rate was 90 mL/min. The elution was performed using a gradient system, and the sample was loaded cyclically onto the column. The mobile phase was initiated for elution, the chromatogram wascollected to observe changes in absorbance. The main peak of the salt exchange was collected and checked for purity by analytical liquid chromatography. The solutions of main peak of salt exchange were combined and concentrated under reduced pressure to obtain a solution of compound A**1** in aqueous acetic acid. The solution was freeze-dried to obtain the pure compound A**1**. ESI-MS (m/z): [M+3H]³⁺:854.5080.

### Example 2

### Synthesis and Characterization of Compounds A2, B1, B2, C1, C2, D1, D2, E1, E2, F1, F2, G1-G26, H1-H34, and 11-116

The synthesis of compounds **A2, B1, B2, C1, C2, D1, D2, E1, E2, F1, F2, G1-G26, H1-H34, and I1-I16** can be carried out by referring to the preparation method in Example 1. In each step, the corresponding protected amino acid raw materials were replaced according to the structure of the product (according to the structure of the compounds, the uppercase letter denotes the corresponding protected L-amino acid, and the lowercase letter denotes the corresponding protected D-amino acid).

**Compound B1:** ESI-MS (m/z): [M+3H]³⁺:868.5246.

**Compound C1:** ESI-MS (m/z): [M+4H]⁴⁺:633.9.

**Compound G1:** ESI-MS (m/z): [M+3H]³⁺:845.1684.

**Compound G3:** ESI-MS (m/z): [M+3H]³⁺:845.1681.

**Compound G5:** ESI-MS (m/z): [M+3H]³⁺:845.1679.

**Compound G7:** ESI-MS (m/z): [M+3H]³⁺:845.1679.

**Compound G9:** ESI-MS (m/z): [M+3H]³⁺:845.1680.

**Compound G11:** ESI-MS (m/z): [M+3H]³⁺: 845.1680.

**Compound H1:** ESI-MS (m/z): [M+5H]⁵⁺: 507.2.

**Compound H3:** ESI-MS (m/z): [M+4H]⁴⁺: 633.9.

**Compound H5:** ESI-MS (m/z): [M+5H]⁵⁺: 510.0.

**Compound H7:** ESI-MS (m/z): [M+5H]⁵⁺: 510.0.

**Compound H9:** ESI-MS (m/z): [M+4H]⁴⁺: 637.4.

**Compound H11:** ESI-MS (m/z): [M+3H]³⁺: 845.1660.

**Compound H17:** ESI-MS (m/z): [M+3H]³⁺: 849.8375.

**Compound H23:** ESI-MS (m/z): [M+3H]³⁺: 854.5190.

**Compound H33:** ESI-MS (m/z): [M+3H]³⁺: 845.4945.

**Compound I1:** ESI-MS (m/z): [M+3H]³⁺: 845.4955.

**Compound I3:** ESI-MS (m/z): [M+3H]³⁺: 845.1671.

**Compound I5:** ESI-MS (m/z): [M+3H]³⁺: 849.8393.

**Compound 17:** ESI-MS (m/z): [M+3H]³⁺: 849.8391.

**Compound 19:** ESI-MS (m/z): [M+3H]³⁺: 854.5104.

**Compound I11:** ESI-MS (m/z): [M+4H]⁴⁺: 630.3.

**Compound 113:** ESI-MS (m/z): [M+3H]³⁺: 849.8387.

**Compound I15:** ESI-MS (m/z): [M+3H]³⁺: 850.1670.

### Example 3

### Synthesis and Characterization of Compound 1

YGrKKRrQrRkLSSIESDV **1**

### 4) Synthesis and Characterization of Compound 1 Peptide Resin

**wherein, the structure of compound 1 peptide resin is: Boc-Tyr(tBu)-Gly-D-Arg(Pbf)-Lys(Boc)-Lys(Boc)-Arg(Pbf)-D-Arg(Pbf)-Gln(Trt)-D-Arg(Pbf)-Arg(Pbf)-D-Lys(Boc)-Leu-Ser(tBu)-Ser(tBu)-Ile-Glu(OtBu)-Ser(tBu)-Asp(OtBu)-Val-Wang resin**
The Fmoc-Val-Wang resin was used as the starting resin and sequentially coupled with the protected amino acids listed in Table 2 through the removal of the Fmoc protection and the coupling reaction, to prepare Boc-Tyr(tBu)-Gly-D-Arg(Pbf)-Lys(Boc)-Lys(Boc)-Arg(Pbf)-D-Arg(Pbf)-Gln(Trt)-D-Arg(Pbf)-Arg(Pbf)-D-Lys(Boc)-Leu-Ser(tBu)-Ser(tBu)-Ile-Glu(OtBu)-Ser(tBu)-Asp(OtBu)-Val-Wang resin (For amino acids listed in Table 2, according to the structure of compound 1, uppercase letters denote the corresponding protected L-amino acids, and lowercase letters denote the corresponding protected D-amino acids).

**Table 3**

| Coupling Peptide Order n = | | Protected Amino Acid |
|---|---|---|
| | 2 | Fmoc-Asp(OtBu) |
| | 3 | Fmoc-Ser(tBu) |
| | 4 | Fmoc-Glu(OtBu) |
| | 5 | Fmoc-Ile |
| | 6 | Fmoc-Ser(tBu) |
| | 7 | Fmoc-Ser(tBu) |
| | 8 | Fmoc-Leu |
| | 9 | Fmoc-D-Lys(Boc) |
| | 10 | Fmoc-Arg(Pbf) |
| | 11 | Fmoc-D-Arg(Pbf) |
| | 12 | Fmoc-Gln(Trt) |
| | 13 | Fmoc-D-Arg(Pbf) |
| | 14 | Fmoc-Arg(Pbf) |
| | 15 | Fmoc-Lys (Boc) |
| | 16 | Fmoc-Lys (Boc) |
| | 17 | Fmoc-D-Arg(Pbf) |
| | 18 | Fmoc-Gly |
| | 19 | Boc-Tyr(tBu) |

The specific steps are as follows:

### a) Coupling of the 2nd amino acid

0.03 mol of the 2nd protected amino acid and 0.03 mol HOBt were dissolved in an appropriate amount of DMF. Additionally, 0.03 mol DIC was slowly added to the solution of protected amino acid in DMF with stirring, and the mixture was stirred for reaction at room temperature for 30 minutes to obtain the activated protected amino acid solution, for future use.

0.01 mol of Fmoc-Val-Wang resin (substitution value of ~0.5 mmol/g) was taken and deprotected using a 20% PIP/DMF solution for 25 minutes, followed by washing and filtering to obtain the Fmoc-deprotected resin.

The solution of the 2nd protected amino acid upon activated was added to the Fmoc-deprotected resin for the coupling reaction for 120-300 minutes. The resin was filtered and washed to obtain a resin containing 2 protected amino acids.

### b) Coupling of the 3rd to 19th amino acids

Using the same method described above, the aforementioned corresponding 3rd to 19th protected amino acids or fragments described above were sequentially coupled to obtain the compound 1 peptide resin.

### 5) Preparation of Crude Compound 1

To the compound 1 peptide resin obtained in step 1) was added a cleavage cocktail with the volume ratio of TFA:Water: EDT = 95:5:5 (10 mL per gram of resin). The mixture was mixed well and stirred for reaction at room temperature for 3 hours. The reaction mixture was filtered by a sand funnel, and the filtrate was collected. The resin was washed three times with a small amount of TFA. The combined filtrates were concentrated under reduced pressure. Anhydrous ethyl ether was added to precipitate and the precipitate was then washed three times with anhydrous ethyl ether and dried under reduced pressure at 35-45°C to afford crude compound 1.

### 6) Preparation of Pure Compound 1

The crude compound 1 obtained in step 2) was dissolved in 10% acetic acid solution, and the resulting solution was filtered through 0.45 µm mixed microporous filter membrane and purified for future use.

The purification was carried out using high performance liquid chromatography. The chromatographic packing material for purification was 10 µm reversed-phase C18, and two mobile phase systems were used for the purification alternately. The first mobile phase system was 0.1% TFA in water-0.1% TFA in acetonitrile, and the second mobile phase system was 50 mmol ammonium acetate in water-acetonitrile. The 77 mm*250 mm column flow rate was 90 mL/min. The elution was performed using a gradient system, and the sample was loaded cyclically for purification. The crude solution was loaded onto the column. The mobile phase was initiated for elution, and the main peak was collected. The obtained solution was evaporated to remove acetonitrile, and then filtered through a 0.45 µm membrane to obtain the concentrated solution of the purified compound 1 intermediate;

The salt exchange was carried out using high performance liquid chromatography. The mobile phase system was 1% acetic acid in water-acetonitrile, and the chromatographic packing material for purification was 10 µm reversed-phase C18. The 77 mm*250 mm column flow rate was 90 mL/min. The elution was performed using a gradient system, and the sample was loaded cyclically onto the column. The mobile phase was initiated for elution, the chromatogram was collected to observechanges in absorbance. The main peak of the salt exchange was collected and checked for purity by analytical liquid chromatography. The solutions of main peak of salt exchange were combined, and concentrated under reduced pressure to obtain a solution of compound 1 in aqueous acetic acid. The solution was freeze-dried to obtain the pure compound 1. ESI-MS (m/z): [M+3H]³⁺: 788.1.

### Example 4

### Synthesis and Characterization of Compounds 2-42

The synthesis of compounds 2-42 can be carried out by referring to the preparation method in Example 1. In each step, the corresponding protected amino acid raw materials were replaced according to the structure of the product (according to the structure of the compounds, the uppercase letter denotes the corresponding protected L-amino acid, and the lowercase letter denotes the corresponding protected D-amino acid).

The structure of compound **15** is: YGrKKRrErRRkLSSIESDV; the mass spectrometry data is: ESI-MS (m/z): [M+4H]⁴⁺: 630.6.

### Example 5

### Preparation and characterization of compound A3

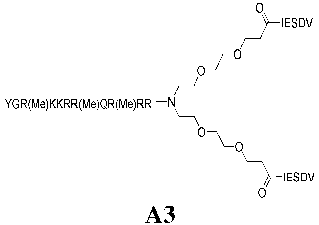

### 1) Synthesis of the Ns-N[(PEG)2-COOH]2 linker:

2-Chlorotrityl chloride resin (3 mmol, 1.90 g) was rinsed and swollen in DMF (for about 20 minutes). Then, a DMF solution (about 8 mL) containing Fmoc-NH-PEG2-CH₂CH₂COOH (2 mmol, 800 mg) was added to the drained resin, followed by the addition of DIPEA (10 mmol, 1.75 mL) and shaking for 60 minutes. Methanol (1 mL) was then added and further shaken for more than 5 minutes to complete the loading of Fmoc-NH-PEG2-CH₂CH₂COOH onto the resin. The loaded resin was drained, and then was rinsed with DMF (10-15 flow rinses, about 10 mL each). The Fmoc groups were removed with DMF containing 20% piperidine for 5 min, and then the resin was rinsed with DMF (10 mL), and further rinsed with DMF and THF for 15 minutes.

The resin was swollen in DIPEA (2.1 mL) and THF (8 mL) for 15minutes, then DCM (5 mL) containing o-nitrobenzenesulfonyl chloride (NsCl, 8 mmol, 1.78 g) was added slowly, and the mixture was stirred for reaction. After 4 hours, the resin was drained and rinsed with THF, MeOH, DCM and THF in order.

The resin was treated with THF (5 mL) containing triphenylphosphine (10 mmol, 2.625 g) and THF (5 mL) containing HO-PEG₂-CH₂CH₂COOtBu (10 mmol, 2.34 g), followed by the addition of diisopropyl azodicarboxylate (DIAD, 10 mmol, 2.02 g) dropwise and shaking for reaction for 1 h. Then the resin was thoroughly rinsed with THF and DCM and dried under vacuum. The resin was treated with a cocktail of TFA/triisopropylsilane/water (90/5/5, 20mL) for 2.5 hours. The resin was filtered and rinsed with TFA and DCM. The rinsate and filtrate were combined and concentrated. The concentrated residue was dissolved in water/acetonitrile (75/25,100 mL) and lyophilized to obtain the Ns-N[(PEG)₂-COOH]₂ linker, which was used directly in subsequent synthesis.

### 2) Synthesis of Ile-Glu(OtBu)-Ser(tBu)-Asp(OtBu)-Val-Wang Resin:

Based on Fmoc-based solid-phase synthesis peptide chemistry and using pre-loaded Fmoc-Val-Wang Resin (0.6-0.7 mmol/g, 100-200 mesh), HBTU/DIPEA as coupling agent and anhydrous DMF as solvent, the resin-containing peptide sequence Ile-Glu(OtBu)-Ser(tBu)-Asp(OtBu)-Val-Wang Resin (IESDV) was synthesized. The resin/Fmoc-amino acid/HBTU/DIPEA equivalent of 1/4/3.9/8 was used for each coupling, which were qualitatively evaluated using the ninhydrin test. The Fmoc protecting group was removed using a DMF solution containing 20% piperidine.

### 3) Synthesis of the NPEG(4)-based Dimer:

The Ns-N[(PEG)₂-COOH]₂ linker (0.025 mmol) was activated using HBTU (0.05 mmol) and DIPEA (0.1 mmol) and added to a DMF (4 mL) containing Ile-Glu(OtBu)-Ser(tBu)-Asp(OtBu)-Val-Wang Resin (0.25 mmol), and the mixture was shaken for reaction for 4 hours. DMF (2 mL) containing DBU (0.5 mmol) was added, and DMF(2 mL) containing mercaptoethanol (0.5 mmol) was added, and the mixture was shaken for reaction for 30 muiutes to remove the Ns protecting group. The resin was drained, rinsed with DMF, and repeatedly treated with mercaptoethanol/DBU once. The resin was then rinsed using DMF, DCM, MeOH, and DCM in order to obtain the NPEG(4)-based dimer:

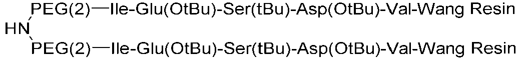

### 4) Synthesis of Compound A3:

Based on Fmoc-based solid-phase synthesis peptide chemistry and using the NPEG(4)-based dimer, HBTU/DIPEA as coupling agent and anhydrous DMF as solvent, the resin-containing peptide sequence: Boc-Tyr(tBu)-Gly-Arg(Me,Pbf)-Lys(Boc)-Lys(Boc)-Arg(Pbf)-Arg(Me,Pbf)-Gln(Trt)-Arg(Me,Pbf)-Arg(Pbf)-Arg(Pbf)-{N,N-Di(PEG2-Ile-Glu( OtBu)-Ser(tBu)-Asp(OtBu)-Val-Wang Resin)} was synthesized. (according to the structure of the compound A3, the uppercase letters denote the corresponding protected L-amino acid, and the lowercase letters denotes the corresponding protected D-amino acid) wherein, a structural formula of R(Me) is: 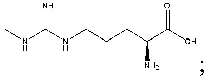 a raw material of Arg(Me,Pbf) is Fmoc-Arg(Me,Pbf), and the specific structural formula is:

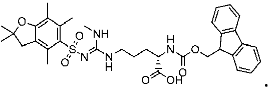

The resin/Fmoc-amino acid/HBTU/DIPEA equivalent of 1/4/3.9/8 was used for each coupling, which were qualitatively evaluated using the ninhydrin test. The Fmoc protecting group was removed using a DMF solution containing 20% piperidine.

To the resin was added a cleavage cocktail with the volume ratio of TFA:Water: EDT = 95:5:5 (10 mL per gram of resin). The mixture was mixed well and stirred for reaction at room temperature for 3 hours. The reaction mixture was filtered by a sand funnel, and the filtrate was collected. The resin was washed three times with a small amount of TFA. The combined filtrates were concentrated under reduced pressure. Anhydrous ethyl ether was added to precipitate and the precipitate was washed three times with anhydrous ethyl ether and dried under reduced pressure at 35-45°C to afford crude compound A3. The crude compound **A3** was purified using high performance liquid chromatography. The mobile phase system was 1% acetic acid in water-acetonitrile, and the chromatographic packing material for purification was 10 µm reversed-phase C18. The elution was performed using a gradient system, and the sample was loaded cyclically onto the column. The mobile phase was initiated for elution, and the chromatogram was collected to observe changes in absorbance. The main peak of the salt exchange was collected and checked for purity by analytical liquid chromatography. The solutions of main peak of salt exchange were combined and concentrated under reduced pressure to obtain an aqueous acetic acid solution. The solution was freeze-dried to obtain the pure compound A3.

### Example 6

### Synthesis and characterization of compounds A4-A8, B3-B8, C3-C8, D3-D8, E3-E8, F3-F8, G27-G78, H35-H102, and 117-128

The synthesis of the compounds **A4-A8, B3-B8, C3-C8, D3-D8, E3-E8, F3-F8, G27-G78, H35-H102, and I17-I28** can be carried out by referring to the preparation method of Example 3. In each step, the corresponding protected amino acid raw materials were replaced according to the structure of the product (according to the structure of the compounds, the uppercase letter denotes the corresponding protected L-amino acid, and the lowercase letter denotes the corresponding protected D-amino acid).

### Example 7

### Synthesis and characterization of Compound 43

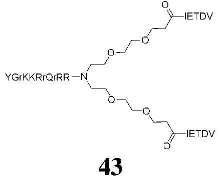

### 1) Synthesis of the Ns-N[(PEG)2-COOH]2 linker:

2-Chlorotrityl chloride resin (3 mmol, 1.90 g) was rinsed and swollen in DMF (for about 20 minutes). Then, a DMF solution (about 8 mL) containing Fmoc-NH-PEG2-CH₂CH₂COOH (2 mmol, 800 mg) was added to the drained resin, followed by the addition of DIPEA (10 mmol, 1.75 mL) and shaking for 60 minutes. Methanol (1 mL) was then added and further shaken for more than 5 minutes to complete the loading of Fmoc-NH-PEG2-CH₂CH₂COOH onto the resin. The loaded resin was drained, and then was rinsed with DMF (10-15 flow rinses, about 10 mL each). The Fmoc groups were removed with DMF containing 20% piperidine for 5 min, and then the resin was rinsed with DMF (10 mL), and further rinsed with DMF and THF for 15 minutes.

The resin was swollen in DIPEA (2.1 mL) and THF (8 mL) for 15minutes, then DCM (5 mL) containing o-nitrobenzenesulfonyl chloride (NsCl, 8 mmol, 1.78 g) was added slowly, and the mixture was stirred for reaction. After 4 hours, the resin was drained and rinsed with THF, MeOH, DCM and THF in order.

The resin was treated with THF (5 mL) containing triphenylphosphine (10 mmol, 2.625 g) and THF (5 mL) containing HO-PEG₂-CH₂CH₂COOtBu (10 mmol, 2.34 g), followed by the addition of diisopropyl azodicarboxylate (DIAD, 10 mmol, 2.02 g) dropwise and shaking for reaction for 1 h. Then the resin was thoroughly rinsed with THF and DCM and dried under vacuum. The resin was treated with a cocktail of TFA/triisopropylsilane/water (90/5/5, 20mL) for 2.5 hours. The resin was filtered and rinsed with TFA and DCM. The rinsate and filtrate were combined and concentrated. The concentrated residue was dissolved in water/acetonitrile (75/25,100 mL) and lyophilized to obtain the Ns-N[(PEG)₂-COOH]₂ linker, which was used directly in subsequent synthesis.

### 2) Synthesis of Ile-Glu(OtBu)-Thr(tBu)-Asp(OtBu)-Val-Wang Resin:

Based on Fmoc-based solid-phase synthesis peptide chemistry and using pre-loaded Fmoc-Val-Wang Resin (0.6-0.7 mmol/g, 100-200 mesh), HBTU/DIPEA as coupling agent and anhydrous DMF as solvent, the resin-containing peptide sequence Ile-Glu(OtBu)-Thr(tBu)-Asp(OtBu)-Val-Wang Resin (IESDV) was synthesized. The resin/Fmoc-amino acid/HBTU/DIPEA equivalent of 1/4/3.9/8 was used for each coupling, which were qualitatively evaluated using the ninhydrin test. The Fmoc protecting group was removed using a DMF solution containing 20% piperidine.

### 3) Synthesis of the NPEG(4)-based Dimer:

The Ns-N[(PEG)₂-COOH]₂ linker (0.025 mmol) was activated using HBTU (0.05 mmol) and DIPEA (0.1 mmol) and added to a DMF (4 mL) containing Ile-Glu(OtBu)-Thr(tBu)-Asp(OtBu)-Val-Wang Resin (0.25 mmol), and the mixture was shaken for reaction for 4 hours. DMF (2 mL) containing DBU (0.5 mmol) was added, and DMF (2 mL) containing mercaptoethanol (0.5 mmol) was added, and the mixture was shaken for reaction for 30 muiutes to remove the Ns protecting group. The resin was drained, rinsed with DMF, and repeatedly treated with mercaptoethanol/DBU once. The resin was then rinsed using DMF, DCM, MeOH, and DCM in order to obtain the NPEG(4)-based dimer:

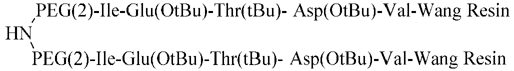

### 4) Synthesis of Compound 43:

Based on Fmoc-based solid-phase synthesis peptide chemistry and using the NPEG(4)-based dimer, HBTU/DIPEA as coupling agent and anhydrous DMF as solvent, the resin-containing peptide sequence: Fmoc-Tyr(tBu)-Gly-D-Arg(Pbf)-Lys(Boc)-Lys(Boc)-Arg(Pbf)-D-Arg(Pbf)-Gln(Trt)-D-Arg(Pbf)-Arg(Pbf)- Arg(Pbf)- { N,N-Di(PEG2-Ile-Gl-
u(OtBu)-Thr(tBu)-Asp(OtBu)-Val-Wang Resin)} was synthesized (according to the structure of the compound 43, the uppercase letter denotes the corresponding protected L-amino acid, and the lowercase letter denotes the corresponding protected D-amino acid).

The resin/Fmoc-amino acid/HBTU/DIPEA equivalent of 1/4/3.9/8 was used for each coupling, which were qualitatively evaluated using the ninhydrin test. The Fmoc protecting group was removed using a DMF solution containing 20% piperidine.

To the resin was added a cleavage cocktail with the volume ratio of TFA:Water: EDT = 95:5:5 (10 mL per gram of resin). The mixture was mixed well and stirred for reaction at room temperature for 3 hours. The reaction mixture was filtered by a sand funnel, and the filtrate was collected. The resin was washed three times with a small amount of TFA. The combined filtrates were concentrated under reduced pressure. Anhydrous ethyl ether was added to precipitate and the precipitate was washed three times with anhydrous ethyl ether and dried under reduced pressure at 35-45°C to afford crude compound 43. The crude compound 43 was purified using high performance liquid chromatography. The mobile phase system was 1% acetic acid in water-acetonitrile, and the chromatographic packing material for purification was 10 µm reversed-phase C18. The elution was performed using a gradient system, and the sample was loaded cyclically onto the column. The mobile phase was initiated for elution, and the chromatogram was collected to observe changes in absorbance. The main peak of the salt exchange was collected and checked for purity by analytical liquid chromatography. The solutions of main peak of salt exchange were combined, and concentrated under reduced pressure to obtain an aqueous acetic acid solution. The solution was freeze-dried to obtain the pure compound 43. ESI-MS (m/z): [M+5H]⁵⁺: 599.9.

### Example 8

### Synthesis and Characterization of Compounds 44-134

The synthesis of compounds 44-134 can be carried out by referring to the preparation method of Example 3. In each step, the corresponding protected amino acid raw materials were replaced according to the structure of the product (according to the structure of the compounds, the uppercase letter denotes the corresponding protected L-amino acid, and the lowercase letter denotes the corresponding protected D-amino acid).
**Compound 44:** ESI-MS (m/z): [M+3H]³⁺: 989.5544;
**Compound 65:** ESI-MS (m/z): [M+3H]³⁺: 999.2264.

### Example 9: Determination of Affinity for the PDZ Domain of PSD-95 by Fluorescence Polarization (FP) Assay

An *in vitro* affinity assay was performed according to the principle of fluorescence polarization to measure the affinity constants (Kᵢ value) between the compounds obtained in Examples 1-4 and the PDZ1-2 of PSD95. First, the affinity between the 5-FAM-labeled probe (5-FAM-NA-1, molecular weight: 2877.23) and PDZ1-2 was determined by a saturation binding assay, in which PDZ1-2 of gradually increased concentrations were added to of the probe of a fixed concentration (0.5 nM). The assay was conducted in TBS buffer (137 mM NaCl, 20 mM Tris, pH 7.6) in black flat-bottomed 384-well plates (Greiner bio-one, Germany). After 10 minutes of incubation at room temperature, the fluorescence polarization of the samples was measured at excitation/emission values of 485/520 nm through a PHERAstar FSX plate reader (BMG LABTECH, Ortenberg, Germany). The fluorescence polarization value was fitted to the equation Y=Bmax×X(K_{d}+X), where Bmax is the maximum fluorescence polarization value, X is the concentration of PDZ1-2, and Y is the fluorescence polarization value. K_{d} can be obtained directly from the saturation curve, corresponding to the concentration of PDZ1-2 at half-saturation and equal to 1010 nM. The affinity between the non-fluorescent compounds and PDZ1-2 was determined by heterologous competition assay, in which the compounds of gradually increased concentrations were added to probe (0.25 nM) of a fixed concentration and PDZ1-2 (2500 nM) in the same TBS buffer under the conditions described above. The FP value was fited to the general equation: Y=Bottom + (Top-Bottom)/[1+(10 (X-LogIC₅₀*HillSlope))], where X is the logarithm of the peptide concentration. The resulting IC₅₀ values were converted into the competitive inhibition constant, Kᵢ value. All reported values were the average of at least three independent experiments. The results are shown in Table 4.

**Table 4. Results of binding assay of PDZ1-2 (2500 nM) with probe (5-FAM-NA-1) (0.25 nM)**

| Compound | IC₅₀ (µM) |
|---|---|
| **NoNo-42** | 9.71 |
| **Compound B1** | 7.70 |
| **Compound G1** | 4.40 |
| **Compound G3** | 5.20 |
| **Compound G5** | 5.23 |
| **Compound G7** | 3.96 |
| **Compound G9** | 6.32 |
| **Compound H3** | 13.54 |
| **Compound I13** | 6.33 |
| **Compound I15** | 7.89 |
| **Compound 1** | 14.92 |
| **Compound 15** | 11.30 |

According to the results of Table 4, it can be seen that the compounds of the present invention have excellent affinity for PDZ1-2, and comparable or superior affinity for PDZ1-2 compared to NoNo-42.

### Example 6: Determination of Affinity for the PDZ Domain of PSD-95 by Fluorescence Polarization (FP) Assay

An *in vitro* affinity assay was performed according to the principle of fluorescence polarization to measure the affinity constants (Kᵢ value) between the compounds obtained in Examples 1-8 and the PDZ1-2 of PSD95. First, the affinity between the 5-FAM-labeled probe (5-FAM-Gly-N,N-Di(PEG₂-Ile-Glu-Thr-Ala-Val-OH, molecular weight: 1867.8), or 5-FAM-Gly-N,N-Di(PEG2-Ile-Glu-Ser-Asp-Val-OH) and PDZ1-2 was determined by a saturation binding assay, in which PDZ1-2 of gradually increased concentrations were added to the probe of a fixed concentration (0.5 nM). The assay was conducted in TBS buffer (137 mM NaCl, 20 mM Tris, pH 7.6) in black flat-bottomed 384-well plates (Greiner bio-one, Germany). After 10 minutes of incubation at room temperature, the fluorescence polarization of the samples was measured at excitation/emission values of 485/520 nm through a PHERAstar FSX plate reader (BMG LABTECH, Ortenberg, Germany). The fluorescence polarization value was fitted to the equation Y=Bmax×X(K_{d}+X), where Bmax is the maximum fluorescence polarization value, X is the concentration of PDZ1-2, and Y is the fluorescence polarization value. K_{d} can be obtained directly from the saturation curve, corresponding to the concentration of PDZ1-2 at half-saturation and equal to 20.7 ± 0.98 nM. The affinity between the non-fluorescent compounds and PDZ1-2 was determined by heterologous competition assay, in which the compounds of gradually increased concentrations were added to the probe of a fixed concentration (0.5 nM) and PDZ1-2 (20 nM) in the same TBS buffer under the conditions described above. The FP value was fitted to the general equation: Y=Bottom + (Top-Bottom)/[1+(10 (X-LogIC₅₀*HillSlope))], where X is the logarithm of the peptide concentration. The resulting IC₅₀ values were converted into the competitive inhibition constant, Kᵢ value. All reported values were the average of at least three independent experiments.

**Table 5. Results of binding assay of PDZ1-2 (20 nM) with 5-FAM labeled probe (0.5 nM)**

| Compound | IC₅₀(nM) |
|---|---|
| **43** | 11.32 |
| **65** | 8.863 |

According to the results of Table 5, it can be seen that the compounds of the present invention have excellent affinity for the PDZ1-2 domain.

### Example 7: In vitro Plasma Stability Experiment

### (1) Experimental materials

Plasma: Human plasma is commercially available.
Reagents: DMSO (dimethyl sulfoxide), disodium hydrogen phosphate, sodium dihydrogen phosphate, methanol, formic acid, and propranolol (internal standard) are commercially available.
Instrument: AB SCIEX QTRAP 5500+.

### (2) Experiment Methods

5 µL of stock solution (10 mM) was diluted with 495 µL of 0.05 M sodium phosphate buffer to 100 µM of dosing solution. The final concentration of tested drug for incubation was 10 µM.

A certain volume (e.g., 27 µL or 24 µL) of plasma was added into a 96-well plate. An appropriate volume of the internal standard working solution (e.g., 150 µL) was immediately added to precipitate. An appropriate volume of the tested drug dosing solution (e.g., 3 µL of test substance alone, or 3 µL + 3 µL of test substance + rt-PA (final concentration of 0.135 mg/mL)) was added, mixed well, and placed in a 2-8°C refrigerator as the initial 0-hour sample. This was performed in duplicate.

Then, an appropriate amount of plasma (e.g., 27 µL or 24 µL) was added at the designed time points (e.g., 5 min, 15 min, 30 min, 60 min, and 120 min, etc.), and the parallel replicate wells were performed at each time point. The system was placed at 37 °C for 5 min of preincubation, and then an appropriate amount of the tested drug dosing solution was added (e.g., 3 µL of test substance alone, or 3 + 3 µL of test substance + rt-PA (final concentration of 0.135 mg/mL)) was added to initiate the reaction. The reaction was terminated by adding an appropriate amount of internal standard working solution (e.g., 150 µL) at the end of the time, and the mixture was mixed thoroughly.

The mixture was centrifuged at 4000 rpm for 15 min. A certain amount of the supernatant was taken, mixed with a certain amount of water, and then subjected to LC/MS/MS analysis. LC-MS detection conditions were as follows:
Chromatography column: YMC Triart C18, 50*3.0mm, 2.1µm.
Mobile phase: Gradient elution was performed using water (0.1% formic acid)-acetonitrile according to the table below.

| **Time (min)** | **Water (with 0.1% formic acid)** | **Acetonitrile** |
|---|---|---|
| 0 | 95% | 5% |
| 0.6 | 95% | 5% |
| 2.6 | 40% | 60% |
| 3.1 | 40% | 60% |
| 3.11 | 95% | 5% |
| 4 | 95% | 5% |

### (3) Data processing

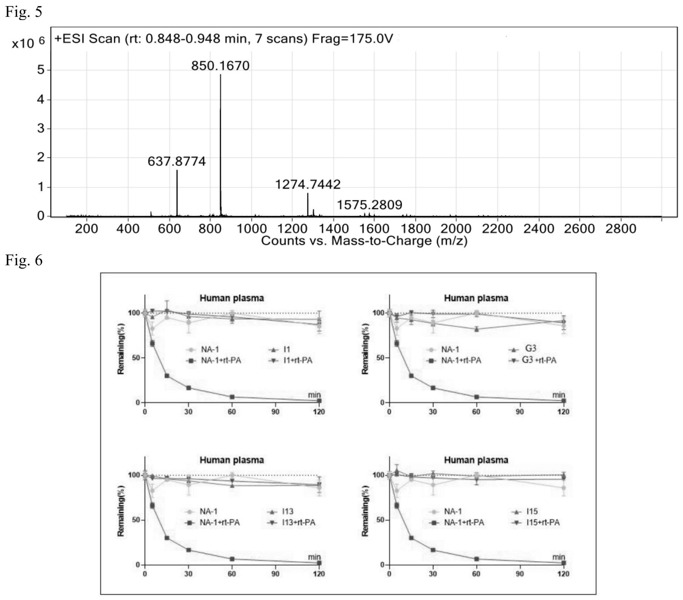

The relative content of drug at each time point was calculated using the initial 0 point as 100%. The half-life of the drug was calculated by plotting "relative drug content" against "incubation time" using GraphPad Prism 5 software. The results are shown in Table 6 and Fig. 6.

**Table 6: Stability results of the compounds of the present invention in human plasma**

| **Test Substance** | **Initial concentration** | **Without tPA** | **With tPA** |
|---|---|---|---|
| | | **T_{1/2} (min)** | **T_{1/2} (min)** |
| | **(µM)** | | |
| **NA-1** | 10 | / | 3.61 |
| **NoNO42** | 10 | / | 413 |
| **Compound G3** | 10 | >2000 | 696 |
| **Compound G7** | 10 | >2000 | 758.8 |
| **Compound G11** | 10 | >2000 | 490 |
| **Compound H3** | 10 | >2000 | >2000 |
| **Compound I1** | 10 | 1117 | 551 |
| **Compound 113** | 10 | 643 | 873 |
| **Compound I15** | 10 | >2000 | 1632 |
| **Compound 15** | 10 | >2000 | >2000 |
| **Compound 43** | 10 | >2000 | 1230 |
| **Compound 65** | 10 | >2000 | 628 |

According to the results of Table 6 and Fig. 6, it can be seen that the compounds of the present invention have excellent plasma stability, even in the presence of tPA.

The structure of NA-1 is: YGRKKRRQRRRKLSSIESDV; and the structure of NoNO42 is: YGrKKRrQrRRkLSSIESDV.

### Example 8: Toxicological Experiment

The compounds of Examples 1-8 were accurately weighed into suitable EP tubes, and the vehicle (saline) was added to formulate a solution of a concentration of 15 mg/mL. The solution was filtered using a 0.2 µm filter for future use.

A total of 18 male SD rats of 6-7 weeks of age were used for test. After the animal quarantine and acclimatization period, the animals were randomly divided into 6 groups according to their body weights measured before grouping, vehicle control group (saline) as well as example compounds groups, 3 animals/group. Using the above prepared solution, the animal was administered intravenously via the tail vein at a dosage of 30 mg/kg and a dosing volume of 2 mL/kg. The dosing volume for each animal was calculated according to the body weight. Administration was performed once daily for 5 consecutive days. After 2 h of administration on day 5, the animals were deeply anesthetized by intraperitoneal injection of sodium pentobarbital. The thoracic cavity was opened, and blood was collected from the abdominal aorta into blood collection tubes containing inert separation gel and coagulant. The serum was collected by centrifugation (4000 rpm, 10 min) after about 1 h of resting. The concentrations of UREA and CREA in the serum were detected using a biochemical analyzer. The percentage change of each example compound relative to the vehicle control group was calculated using the formula: % = (Concentration of example compound - Concentration of vehicle control group) / Concentration of vehicle control group. The results are shown in Table 7:

**Table 7 Percentage change compared to vehicle control group**

| Compound | UREA (mmol/mL) | CREA (µmol/mL) |
|---|---|---|
| **NoNo-42** | 237.9% | 308.8% |
| **Compound 1** | 206.9% | 216.9% |
| **Compound 15** | 88.6% | 90.8% |
| **Compound 65** | 23.7% | -3.3% |
| **Compound H3** | 46.4% | 46.2% |

According to the data in Table 7, it can be seen that the compounds of the present invention have much lower nephrotoxicity and are superior to the positive control drug NoNo-42.

### Example 9

### Tissue Distribution Experiment

The TAMRA-labeled example compounds were dissolved in saline to prepare a solution with a concentration of 600 µM/mL. A 100 uL of the above prepared solution was administered, at a dose of 3 nmol/g, to 6-week-old male Balb/c mice (18-22 g) via tail vein injection. After 45 min of circulation, the animals were deeply anesthetized by intraperitoneal injection of sodium pentobarbital. The thoracic cavity was opened, and the mice were subjected to cardiac perfusion using 0.01 M PBS (pH 7.4), and the brains, kidneys, and livers were collected at the end of perfusion. The harvested tissues were immediately placed on ice and stored at -80°C.

The tissues were homogenized using pre-cooled lysis buffer (tissue weight: lysate = 1:5 (g/mL)) by centrifuging at 4000 rpm for 30 min at 4°C, and were placed on ice for 10 min after the completion of homogenization. The supernatant was transferred to a black 96-well plate with a clear bottom, and the TAMRA-peptide was quantified using a PHERAstar FSX plate reader (BMG LABTECH, Ortenberg, Germany). The excitation/emission wavelengths were set at 540/580 nm. The corresponding peptide concentration in the tissues was calculated using a standard curve (blank tissue mixed with TAMRA-peptide, concentration range of 0 -1000 nM). The results are shown in Table 8.

**Table 8 Tissue distribution of the compounds of the present invention**

| **Tested Compound** | **Mean tissue concentration (nmol/g)** |
|---|---|
| | **Brain** |
| **NA-1** | 2.9115 |
| **NoNo42** | 0.77859 |
| **Compound 1** | 5.9220 |
| **Compound 15** | 4.4515 |

According to the data in Table 8, it can be seen that the distribution of the compounds of the present invention in the brain tissue is significantly superior to that of the control group, which indicates that the compounds of the present invention have a stronger ability to cross the blood-brain barrier.

The structure of NA-1 is: YGRKKRRQRRRKLSSIESDV; and the structure of NoNo42 is: YGrKKRrQrRRkLSSIESDV.

### Example 10

### Preparation of cell-penetrating peptides

Referring to the preparation method of Example 3 or 1, the cell-penetrating peptides TAT-1 to TAT-8 were prepared and the results are shown in Table 9.

**Table 9 Structures and characterization data of cell-penetrating peptides**

| No. | Structure | Mass Spectrometry (ESI-MS (m/z)) | Mass Spectrum |
|---|---|---|---|
| **TAT-1** | YGRKKRRQRRR | [M+4H]⁴⁺: 390.7 | Fig. 12 |
| **TAT-2** | YGrKKRrQrRR | [M+4H]⁴⁺: 390.7 | Fig. 13 |
| **TAT-3** | YGrKKRrQrR | [M+4H]⁴⁺: 351.6 | Fig. 14 |
| **TAT-4** | YGrKKRrErRR | [M+4H]⁴⁺: 390.9 | Fig. 15 |
| **TAT-5** | YGrKKRrQR(Me)RR | [M+3H]³⁺: 525.3328 | Fig. 16 |
| **TAT-6** | YGrKKRrER(Me)RR | [M+3H]³⁺: 525.6612 | Fig. 17 |
| **TAT-7** | YGR*KKRrQR(Me)R R | | |
| **TAT-8** | YGR*KKRrER(Me)RR | | |

### Example 11

### In Vitro Stability Experiment of Cell-Penetrating Peptides

Referring to the method of Example 7 above, the test results are shown in Table 10:

**Table 10: Stability results of the cell-penetrating peptide of the present invention in human plasma**

| **TestSubstance** | **Initial concentration** | **Without tPA** | **With tPA** |
|---|---|---|---|
| | | **T_{1/2}** | **T_{1/2}** |
| | **(µM)** | **(min)** | **(min)** |
| **TAT-1** | 10 | 91 | / |
| **TAT-3** | 10 | 1300 | 1648 |
| **TAT-4** | 10 | 717 | 897 |
| **TAT-5** | 10 | >2000 | 738 |
| **TAT-6** | 10 | 170 | 258 |

According to the results in Table 10, it can be seen that the cell-penetrating peptides (TAT) designed by the present invention all have excellent stability compared to the TAT (TAT-1) of NA-1.

The above examples are preferred embodiments of the present invention, but the embodiments of the present invention are not limited by the above examples. Any other changes, modifications, substitutions, combinations, and simplifications made without departing from the spirit and principles of the present invention should be equivalent replacement methods and are included within the scope of protection of the present invention.

## Claims

1. A PSD-95 domain inhibitor, **characterized by** having a structure shown in formula (I):
(TAT)m-L₁-(L₂)n (Formula I);
wherein, the TAT is selected from the group consisting of YGrKKRrQrR, YGrKKRrErR, YGrKKRrErRR, YGrKKRrQrRR, YGR(Me)KKRR(Me)QR(Me)RR, YGR(Me)₂KKRR(Me)₂QR(Me)₂RR, YGR(Me)KKRrQrRR, YGrKKRR(Me)QrRR, YGrKKRrQR(Me)RR, YGR(Me)KKRrQR(Me)RR, YGrKKRR(Me)QR(Me)RR, YGR(Me)KKRR(Me)QrRR, YGr(Me)KKRrQrRR, YGrKKRrQr(Me)RR, YGrKKRr(Me)QrRR, YGr(Me)KKRr(Me)QrRR, YGr(Me)KKRrQr(Me)RR, YGrKKRr(Me)Qr(Me)RR, YGr(Me)KKRr(Me)Qr(Me)RR, YGR(Et)KKRR(Et)QR(Et)RR, YGRKKRrErRR, YGR(Me)KKRrErRR, YGrKKRrERRr, YGR(Ac)KKRR(Ac)QR(Ac)RR, YGR(Ac)₂KKRR(Ac)_{2Q}R(Ac)₂RR, YGR(Ac)KKRrQrRR, YGR*KKRRQRRR, YGR*KKRrQrRR, YGR*KKRrErRR, YGRKKRR*QRRR, YGrKKRR*QrRR, YGRKKRRQR*RR, YGrKKRrQR*RR, YGR*KKRR*QRRR, YGR*KKRR*QrRR, YGRKKRR*QR*RR, YGrKKRR*QR*RR, YGR*KKRRQR*RR, YGR*KKRrQR*RR, YGR*KKRR*QR*RR, YGrKKRrER(Me)RR, YGrKKRrQR(Me)RR(Me), YGrKKRrQRRR, YGR*KKRrQR(Me)RR and YGR*KKRrER(Me)RR, wherein 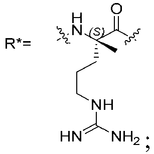
the L₁ is selected from the group consisting of KLSS, K(Me)LSS, kLSS, 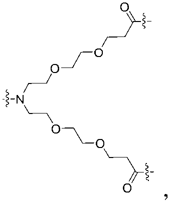 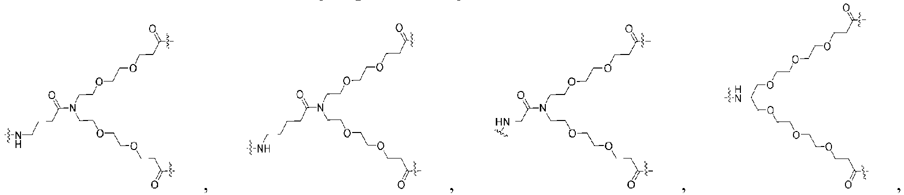 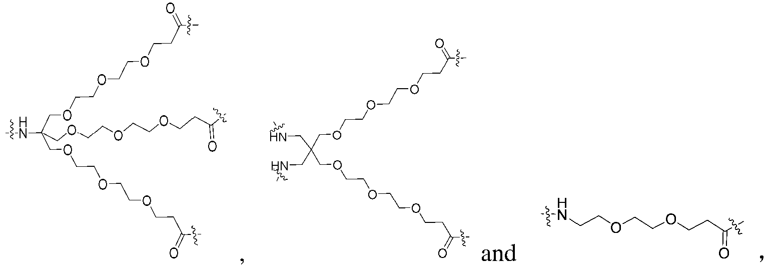 wherein the straight line perpendicular to the wavy line denotes a connection site;
when TAT is YGrKKRrQrRR, L₁ is 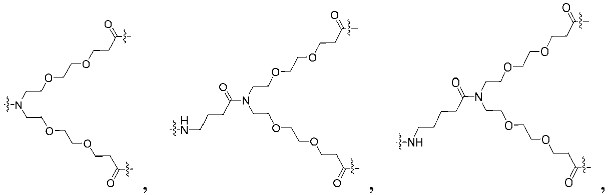 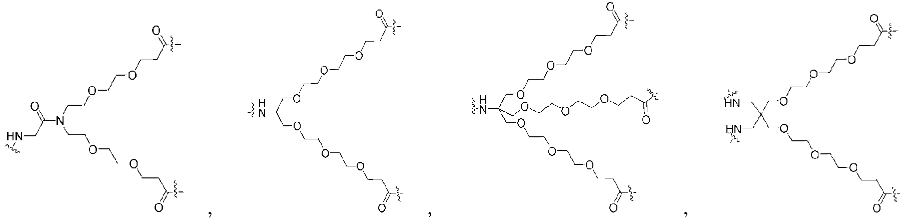 or 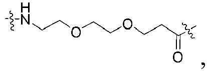 wherein the straight line perpendicular to the wavy line denotes a connection site;
the L₂ is selected from the group consisting of IETDV, IETAV, IESDV, KETLV, KETTV, ISTDV and VETVV;
the m and n are each independently selected from the group consisting of the integers 1, 2 and 3;
for amino acids in the TAT, L₁, and L₂, an uppercase letter denotes an L-amino acid, a lowercase letter denotes a D-amino acid, Me is methyl, Et is ethyl, and Ac is acetyl.

2. The PSD-95 domain inhibitor according to claim 1, **characterized in that**, the TAT is selected from the group consisting of:
YGR(Me)KKRR(Me)QR(Me)RR, YGR(Me)₂KKRR(Me)₂QR(Me)₂RR, YGR(Me)KKRrQrRR, YGrKKRR(Me)QrRR, YGrKKRrQR(Me)RR, YGR(Me)KKRrQR(Me)RR, YGrKKRR(Me)QR(Me)RR, YGR(Me)KKRR(Me)QrRR, YGr(Me)KKRrQrRR, YGrKKRrQr(Me)RR, YGrKKRr(Me)QrRR, YGr(Me)KKRr(Me)QrRR, YGr(Me)KKRrQr(Me)RR, YGrKKRr(Me)Qr(Me)RR, YGr(Me)KKRr(Me)Qr(Me)RR, YGR(Et)KKRR(Et)QR(Et)RR, YGRKKRrErRR, YGR(Me)KKRrErRR, YGrKKRrERRr, YGR(Ac)KKRR(Ac)QR(Ac)RR, YGR(Ac)₂KKRR(Ac)_{2Q}R(Ac)₂RR, YGR(Ac)KKRrQrRR, YGR*KKRRQRRR, YGR*KKRrQrRR, YGR*KKRrErRR, YGRKKRR*QRRR, YGrKKRR*QrRR, YGRKKRRQR*RR, YGrKKRrQR*RR, YGR*KKRR*QRRR, YGR*KKRR*QrRR, YGRKKRR*QR*RR, YGrKKRR*QR*RR, YGR*KKRRQR*RR, YGR*KKRrQR*RR and YGR*KKRR*QR*RR, wherein 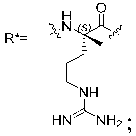
the L₁ is selected from the group consisting of KLSS, kLSS, 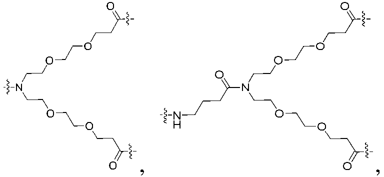 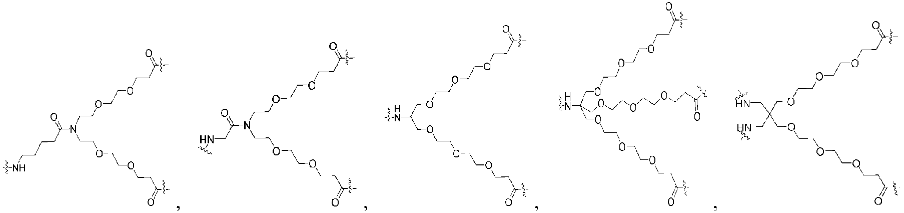 and 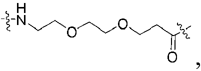 wherein the straight line perpendicular to the wavy line denotes a connection site;
the L₂ is selected from the group consisting of IETDV, IETAV, IESDV, KETLV, KETTV, ISTDV and VETVV;
the m and n are each independently selected from the group consisting of the integers 1, 2 and 3;
for amino acids in the TAT, L₁, and L₂, an uppercase letter denotes an L-amino acid, a lowercase letter denotes a D-amino acid, Me is methyl, Et is ethyl, and Ac is acetyl.

3. The PSD-95 domain inhibitor according to claim 1, **characterized in that**, when TAT is selected from the group consisting of YGrKKRrQrR, YGrKKRrErR and YGrKKRrErRR, the L₁ is selected from the group consisting of KLSS, kLSS, 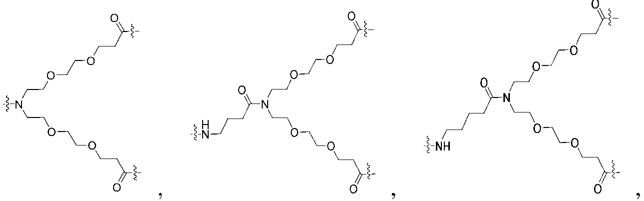 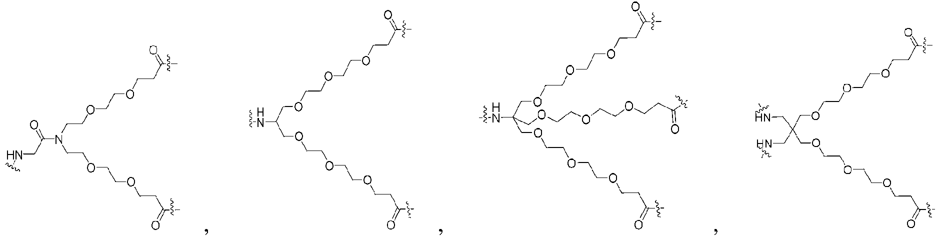 and 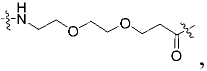 wherein the straight line perpendicular to the wavy line denotes a connection site; when TAT is YGrKKRrQrRR, the L₁ is 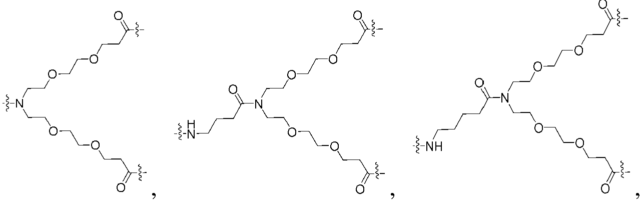 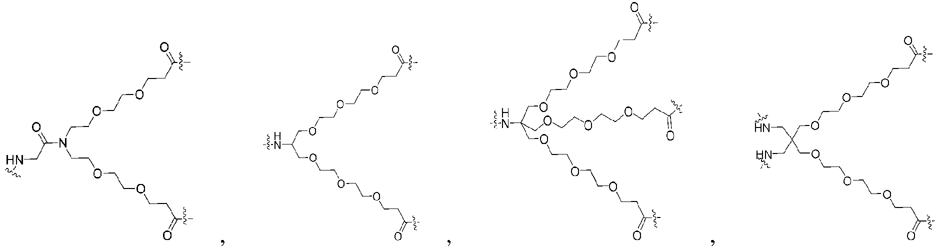 or 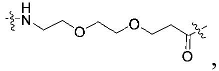 wherein the straight line perpendicular to the wavy line denotes a connection site;
the L₂ is selected from the group consisting of IETDV, IETAV, IESDV, KETLV, KETTV, ISTDV and VETVV;
the m and n are each independently selected from the group consisting of the integers 1, 2 and 3; for amino acids in the TAT, L₁, and L₂, an uppercase letter denotes an L-amino acid, and a lowercase letter denotes a D-amino acid;
preferably, the L₁ is selected from the group consisting of kLSS, 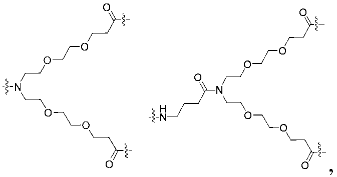 and 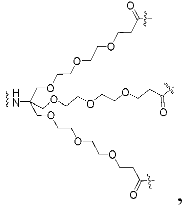 wherein the straight line perpendicular to the wavy line denotes a connection site;
preferably, the L₂ is selected from the group consisting of IETDV and IESDV.

4. The PSD-95 domain inhibitor according to any one of claims 1-3, **characterized in that** the PSD-95 domain inhibitor is selected from a structure shown in Table A, Table B, Table C, Table D, Table E, Table F, Table G, Table H, Table I or Table J.

5. A pharmaceutical composition, **characterized in that** the pharmaceutical composition comprises the PSD-95 domain inhibitor of any one of the preceding claims 1-4 and one or more pharmaceutically acceptable carriers.

6. Use of the PSD-95 domain inhibitor according to any one of claims 1-4 or the pharmaceutical composition according to claim 5 in the preparation of a medicament for the treatment of PSD-95 related diseases.

7. A cell-penetrating peptide, **characterized in that** the cell-penetrating peptide is selected from the group consisting of:
YGRKKRrQrR, YGrKKRRQrR, YGrKKRrQRR, YGRKKRrErRR, YGrKKRrERRr, YGrKKRrErRR, YGRKKRrErR, YGrKKRrERr, YGrKKRrErR, YGR^{#}KKRrQrRR, YGR^{#}KKRR^{#}QrRR, YGR^{#}KKRrQR^{#}RR, YGR^{#}KKRR*QR^{#}RR, YGrKKRR^{#}QrRR, YGrKKRR^{#}QR^{#}RR or YGrKKRrQR^{#}RR, wherein an uppercase letter denotes a L-amino acid, a lowercase letter denotes a D-amino acid, and the symbol # denotes C₁-C₆ alkyl modification, C₂-C₆ acyl modification or a halogen modification;
preferably, the C₁-C₆ alkyl modification is selected from the group consisting of methyl and ethyl; and the C₂-C₆ acyl is selected from acetyl.

8. The cell-penetrating peptide according to claim 7, **characterized in that** the cell-penetrating peptide is selected from the group consisting of the following compounds:
| |
|---|
| YGR*KKRRQRRR, YGR*KKRrQRRR, YGR*KKRRQrRR, YGR*KKRrQrRR |
| YGRKKRR*QRRR, YGrKKRR*QRRR, YGrKKRR*QrRR, YGRKKRR*QrRR |
| YGRKKRRQR*RR, YGrKKRRQR*RR, YGrKKRrQR*RR, YGrKKRrQR*RR |
| YGrKKRrQrR, YGR*KKRrQR(Me)RR, YGR*KKRrER(Me)RR |
| YGR(Me)KKrQrRR, YGrKKRR(Me)QrRR, YGrKKRrQR(Me)RR |
| YGR(Me)KKRrQR(Me)RR, YGrKKRR(Me)QR(Me)RR, YGR(Me)KKRR(Me)QrRR |
| YGr(Me)KKRrQrRR, YGrKKRrQr(Me)RR, YGrKKRr(Me)QrRR |
| YGr(Me)KKRr(Me)QrRR, YGr(Me)KKRrQr(Me)RR, YGrKKRr(Me)Qr(Me)RR |
| YGr(Me)KKRr(Me)Qr(Me)RR, YGR(Et)KKRR(Et)QR(Et)RR |
| YGRKKRrErRR, YGrKKRrERRr, YGrKKRrErRR |
| YGRKKRrErR, YGrKKRrERr, YGrKKRrErR, YGrKKRrQrR |
| YGrKKRrER(Me)RR, YGrKKRrQR(Me)RR(Me) |
| YGR(Me)KKRR(Me)QR(Me)RR, YGR(Me)₂KKRR(Me)₂QR(Me)₂RR |
| YGR(Ac)KKRR(Ac)QR(Ac)RR, YGR(Ac)₂KKRR(Ac)₂QR(Ac)₂RR, YGR(Ac)KKRrQrRR |
wherein,

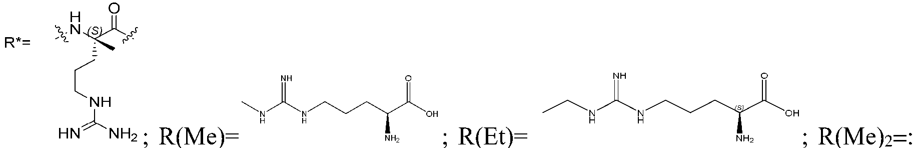

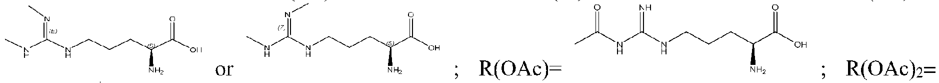

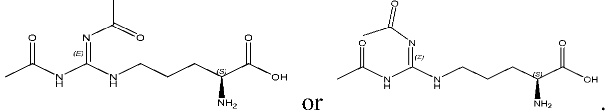

9. A plasmin-resistance peptide, **characterized in that** the plasmin-resistance peptide comprises the cell-penetrating peptide according to any one of claims 7-8, and the cell-penetrating peptide is connected to a functional peptide of the plasmin-resistance peptide directly or via a linker; wherein the functional peptide is selected from the group consisting of one or more of IETDV, IETAV, IESDV, KETLV, KETTV, ISTDV and VETVV.

10. The plasmin-resistance peptide according to claim 9, **characterized in that**, the linker is selected from the group consisting of KLSS, kLSS, 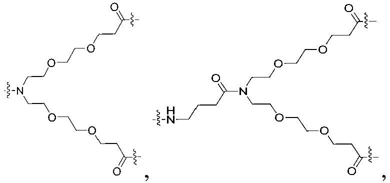 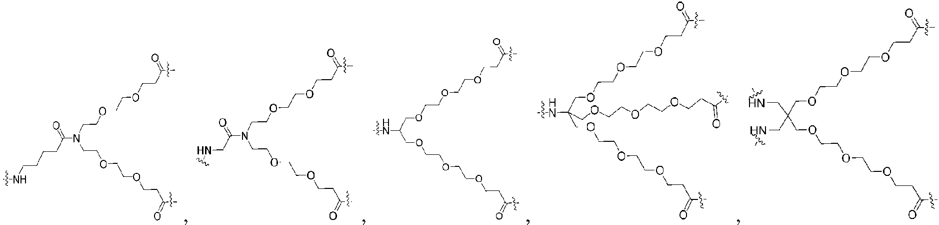 and

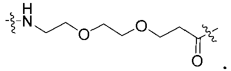

11. Use of the cell-penetrating peptide according to any one of claims 7-10 for delivering a plasmin-resistance peptide.
